# EUROPEAN PATENT SPECIFICATION

(11) **EP 1 781 329 B1**
(45) Date of publication and mention of the grant of the patent: **31.07.2013**
(21) Application number: 05774573.9
(22) Date of filing: 05.08.2005
(51) Int. Cl.: A61N 5/06, A61K 41/00, A61N 5/00, A61K 31/60, A61K 31/327, A61B 18/20, A61B 18/00

(54) **THERAPY DEVICE**
THERAPIEVORRICHTUNG
DISPOSITIF THERAPEUTIQUE

(30) Priority: 06.08.2004 US 599049 P
(43) Date of publication of application: 09.05.2007
(73) Proprietor: Syneron Beauty Ltd., 20692 Yoqneam Illit (IL)
(72) Inventor: Kennedy, John, Guelph, ON N1L 1E7 (CA)
(74) Representative: Nederlandsch Octrooibureau
(86) International application number: PCT/CA2005/001230
(87) International publication number: WO 2006/012752

(56) References cited:
- WO-A1-98/04317
- WO-A1-2004/043543
- US-A- 2 183 726
- US-A- 2 231 092
- US-A- 5 169 384
- US-A- 5 830 211
- US-A- 6 080 127
- US-A1- 2004 147 984
- US-B1- 6 494 900

## Description

### FIELD OF THE INVENTION

This invention relates to therapy devices and in particular to handheld devices and accessories together with compositions .

### BACKGROUND OF THE INVENTION

Therapy using electromagnetic radiation involves the application of energy to biological tissue for the purpose of stimulating biological functions. Although the exact mechanisms of action of such therapy are not known with certainty, the therapy is a well established treatment for a variety of ailments.

Therapy using electromagnetic radiation has been used to treat soft tissue injuries such as capsulitis, bursitis, sprains, strains, hematomas and tendinitis, acute and chronic joint problems such as osteoarthritis, rheumatoid arthritis and ligament and tendon injuries, tendinitis, arthritic pain, chronic pain such as post herpetic neuralgia, chronic back and neck pain, metatarsalgia, trigeminal neuralgia, brachial neuralgia, plantar fisciitis, cellular damage, in vitro fertilization enhancement, stimulation of embryogenesis, soft tissue injury, aging skin, seasonally affected disorder, inflammation, fine lines and wrinkles, mucositis, frozen shoulder, temporomandibular joint diseases and disorders (TMJ) and carpal tunnel syndrome.

Therapy using electromagnetic radiation has also been used to treat non-union and small bone fractures, herpes, apthous ulcers, leg ulcers, dermatitis, wound healing, bums, acute epididymitis, otorhinolaragngology, gynecology, obstetrics, superficial AP stimulation and tonification, cosmetic imperfections, cellulite, and acne, among other things.

Typically, treatment or therapy using electromagnetic radiation involves radiating energy onto or into a patient's skin. The radiation is typically applied at wavelengths either in the visible, ultraviolet, radiofrequency, or the infrared range. A wide variety of radiating energy sources are available and known in the art. The radiating energy sources used in these therapies radiate energy at a wide variety of wavelengths with different wavelengths having been found to be useful depending on the ailment being treated. Acne vulgaris is one of the world's most common skin conditions and results from blockage, bacterial colonization and inflammation of the sebaceous follicles. The main cause of acne stems from an abnormally high amount of bacteria, mainly propionibacterium acnes (P. acnes), resulting in inflammatory acne.

Acne affects between 85-100% of young adults up to the age of 24 years and up to 50% of adults 25 and older. It usually appears on the face, chest, back and limbs and can produce life-long scars, both emotionally and physically. In the United States alone over 17,000,000 people actively seek acne treatment on an ongoing basis. These treatments consist of professionally prescribed pharmaceuticals, cosmeceuticals and invasive skin resurfacing. The P. acnes bacteria has developed up to 80% resistance to antibiotics commonly used to treat acne in the past.

P. acnes absorbs light from the ultraviolet region to about 430 nm, and also absorbs light at about 630 nm. Blue light phototherapy works for a majority of patients with P. Acne vulgaris. The bacteria is made up of an endogenous porphyrin which is a naturally occurring photosensitizer. This photosensitizer absorbs the blue light energy between about 405 to about 425 nanometers and forms a singlet oxygen which simply destroys the bacteria cell. No systemic drugs with their potential side effects and invasive procedures requiring long healing times are necessarily used. For example, radiating energy sources having a peak wavelength of about 415 nm and a bandwidth of about 20 nm have been found particularly useful in the treatment of acne. Peak wavelengths of about 630 nm have also been useful in this regard.

Other examples of electromagnetic radiation useful for treatments include radiation at wavelengths of about 800-810 nm for leg vein and hair removal, wart treatments, hair growth stimulation and tattoo removal, wavelengths of about 1064 nm for skin peel and hair reduction, and of about 574 nm for wrinkle reduction. Varying treatment regimens of pulsing wave (PW) or continuous wave (CW) light, at varying energy levels, are known in the art. Typically, these treatments utilize wavelengths between about 250 and about 2000 nm.

Hand-held therapy devices for delivering electromagnetic radiation are known in the art, however, they are quite expensive and typically limited to one specific use (and one specific wavelength spectrum).

Accordingly, there is a need for a device for delivering radiation that is flexible enough to provide a variety of treatment regimens and wavelength spectrum, so that the device can be used to treat a variety of ailments. There is also a need for compositions and treatment methods that are useful with such devices.
The US patent application no. 2004/147984 A1 discloses a light therapy device having a head with a faceplate for application to the skin, an energy source within the head, and a material dispensing system.

### SUMMARY OF THE INVENTION

The invention is as defined in the appended claims.

According to one aspect of the invention there is provided a therapy device comprising an energy source for emitting a desired wavelength of electromagnetic radiation, and a material dispensing system disposed on the device for dispensing a desired material for use with the device. The dispensing system can be manually operated and can comprise a control mechanism for controlling the energy source and the material dispensing system. The control mechanism can include a processor and a storage device; the storage device being operable to maintain regimen data; the processor being operable to control the energy source and the material dispensing system in accordance with the regimen data. The storage device can be removable. The control mechanism can operable to control the emission of radiation from the energy source at a power level and for a period of time in accordance with the regimen data. The device can further comprise an interface operable to conduct communications with a computing device for updating the regimen data. The device can also further comprise an interface operable to conduct communications with a computer network having at least one server for updating the regimen data. The device can be further operable to maintain, in the storage device, logging data representing a usage of the therapy device; the device further comprising an interface operable to conduct communications with a computing device uploading the logging data.

According to another aspect of the invention, the energy source can be a light source. The energy source can be selected from the group consisting of electrodeless lamps, microwaves, fluorescent tube, quartz halogen lamp, arc lamp, laser, laser diode, and light emitting diode. The energy source can have at least one peak wavelength selected from about 410, 415, 580, 630, 660, 663, 680, 800, 810, 820, 830, 840, 850, and 900 nm. The energy source can comprise at least one peak wavelength having a band width of about 40 nm, more preferably 20 nm. The energy source can have a peak wavelength of about 415 nm and a bandwidth of about 20 nm. It can operate continuously or in a pulsed manner when activated.

According to another aspect of the invention, the energy source is housed within a head having a removable faceplate or the faceplate may be essentially permanently attached to the head, or the faceplate is integral to the structure of the head. The faceplate can include an extension that is functionally connected to the head.

According to another aspect of the invention, the dispensing system of the device is adapted to receive a removable container for containing the material. The container can have an identifier that is readable by the device.

According to another aspect of the invention, the device further comprises an energy reflective layer disposed on the head.

According to another aspect of the invention, the therapy device further comprises a temperature sensing device for delivering temperature data to the control mechanism. The control mechanism can adjust the energy source when the received temperature data is outside pre-specified limits. The temperature sensing device can be a solid state thermistor.

Another aspect of the invention provides a container adapted to contain a material and adapted for housing within a therapy device for delivering electromagnetic radiation. The container can further comprise a material with an index of refraction of approximately 1.5. The container can further comprise a material containing a medicament, active ingredient or supplement.

According to another aspect of the invention, the container can comprise a medicament for a treatment selected from the group consisting of anti-aging treatment, photorejuvenation treatment, cosmetic treatment, acne treatment, cancer treatment, cellulite treatment, and photodamage treatment.

According to another aspect of the invention, the container further comprises an indicator adapted to be read by a reader disposed within a therapy device.

Another aspect of the invention provides a material for use with treatment using electromagnetic radiation. The material can comprise at least one of a medicament, active ingredient, or supplement and can have an index of refraction of approximately 1.5. The medicament, active ingredient, or supplement can be selected from the group consisting of aloe, Vitamin E, a hydration agent, Vitamin C, Vitamin D, Vitamin A, Vitamin K, Vitamin F, Retin A (Tretinoin), Adapalene, Retinol, Hydroquinone, Kojic acid, a growth factor, echinacea, an antibiotic, an antifungal, an antiviral, a bleaching agent, an alpha hydroxy acid, a beta hydroxy acid, salicylic acid, antioxidant triad compound, a seaweed derivative, a salt water derivative, algae, an antioxidant, a phytoanthocyanin, phthalocyanine, a phytonutrient, plankton, a botanical product, a herbaceous product, a hormone, lanolin, an enzyme, a mineral, a genetically engineered substance, a cofactor, a catalyst, an antiaging substance, insulin, trace elements (including ionic calcium, magnesium, etc.), Coenzyme Q10, minerals, minoxidil, a dye, a natural or synthetic melanin, a metalloproteinase inhibitor, proline, hydroxyproline, an anesthetic substance, benzoyl peroxide, amino levulinic acid, chlorophyll, bacteriachlorophyll, neutraceuticals, cosmaceuticals, copper chlorophyllin, chloroplasts, carotenoids, phycobilin, rhodopsin, anthocyanin, and derivatives, subcomponents, immunological complexes and antibodies directed towards any component of the target skin structure or apparatus, and analogs of the above items both synthetic and natural, as well as combinations thereof.

Another aspect of the invention provides that the medicament, active ingredient, or supplement comprised by the material is for the treatment selected from a group consisting of anti-aging treatment, photorejuvenation treatment, cosmetic treatment, acne treatment, cancer treatment, cellulite treatment, and photodamage treatment.

Another aspect of the invention provides a method of treating a condition with a medicament, active ingredient, or supplement in combination with the device and material of the invention.

Another aspect of the invention provides for the use of the device of the invention for the treatment of an ailment selected from the group consisting of arthritic pain, chronic pain, carpel tunnel syndrome, cellular damage, soft tissue injury, acne, TMJ, diabetic neuropathy, neuralgia, aging skin, seasonally affected disorder, inflammation, fine lines and wrinkles, mucositis, psoriasis, rosacia, eczema, oral candida, oral cancer, cellulitis, and wounds.

another aspect of the invention provides for the use of the device and meterial of the invention for the treatment of acne wherein the energy source emits electromagnetic radiation in the range of about 380 to about 460 nm, more preferably in the range of about 395 to about 430 nm, and even more preferably in the range of about 405 to about 425 nm and/or in the range of about 460 to about 1000 nm, more preferably in the range of about 550 to about 900 nm, and even more preferably in the range of about 600 to about 850 nm. The energy source can emit electromagnetic radiation at about 630 nm.

Another aspect of the invention provides for the use of the device and material of the invention for photorejuvenation therapy wherein the energy source emits electromagnetic radiation in the range of about 500 nm to about 1000 nm, more preferably in the range of about 550 nm to about 900 nm and even more preferably in the range of about 570 nm to about 850 nm. The energy source can emit electromagnetic radiation selected from the group consisting of about 580 nm, 630 nm, 633 nm, 660 nm, 830 nm, 900 nm, and combinationations thereof.

Another aspect of the invention provides for the use of the device and material of the invention for the treatment of cellulite wherein the energy source emits electromagnetic radiation including dominant emissions in the range of 500 nm to 1000 nm, more preferably in the range of 550 nm to 800 nm and even more preferably in the range of 570 nm to 850 nm. The energy source can emit laser energy or LED energy at about 810 nm. The energy source can emit a combination of radiofrequency and infrared radiation.

According to another aspect of the invention, the device and materials disclosed herein are used for improving the appearance of the skin of a user.

According to another aspect of the invention, there is provided a faceplate comprising a substrate for supporting an energy source, at least one aperture in the substrate for passing a material therethrough, and a mount for attaching the substrate to a body; the body housing a control mechanism for controlling the energy source and a container for storing the material. The faceplate can further comprise a connector disposed within the substrate that communicates with the control mechanism when mount is attached to the body. The connector can transmit power from a power source in the body to the energy source. The faceplate comprises an identification device that communicates the identity of the faceplate to the control mechanism and/or a memory storage device for storing regimen data.

Another aspect of the invention provides that the faceplate further comprises an outer surface affixed generally parallel to the substrate with the energy source disposed therebetween, the outer surface defining at least one aperture for passing the material therethrough.

According to another aspect of the present invention, there is provided a method for controlling a therapy device having an energy source for emitting radiation, a material dispensing system for dispensing a material and a controller that is operably connected to the energy source and the material dispensing system; the method comprising the steps of (i) receiving, at the controller, regimen data representing a treatment regimen, and (ii) controlling the energy source and the material dispensing system in accordance with the regimen data. The regimen data can be a set of data maintained in a database disposed in the device. The controlling step can include controlling the dispensing of the material from the material dispensing system. The controlling step can include the steps of (i) controlling radiation emission from the energy source at a power level and for a period of time in accordance with the regimen, (ii) receiving temperature data from the device during the time period, and (iii) adjusting the radiation emission when the temperature data is outside a set of limits specified by the regimen data. The energy source can include an identifier for specifying the type of the energy source. The material dispensing system can include an identifier for specifying the type of the material.

According to another aspect of the invention, the therapy device includes an interface operable to conduct communications with a computing device and the method further comprises the steps of (i) storing logging data representing a usage of the therapy device, (ii) establishing a link with a computing device, and (iii) transmitting the logging data to the computing device.

### BRIEF DESCRIPTION OF THE DRAWINGS

Figure 1 shows a front phantom view of a device in accordance with one aspect of the present invention.

Figure 2 shows a front view of a removable container used in the device of Figure 1.

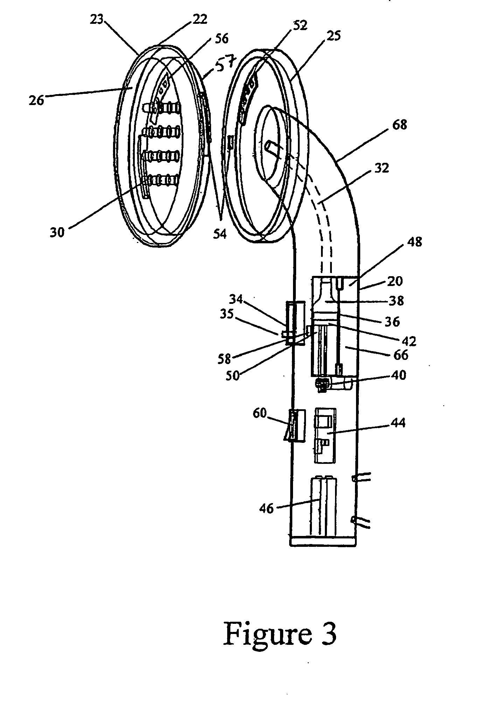

Figure 3 shows an exploded side phantom view of the device of Figure 1.

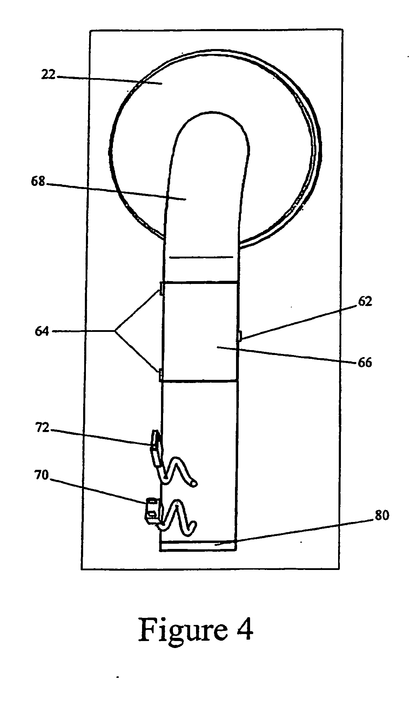

Figure 4 shows a back view of the device of Figure 1.

Figure 5 shows a device connected to a database server through the internet in accordance with another aspect of the present invention.

Figure 6 shows a computer flow chart detailing processor steps for the device of Figure 1.

Figure 7 is a cross-sectional view of the device shown in Figure 1 and shows a temperature sensing device in accordance with one aspect of the present invention.

Figure 8 is a block diagram showing electronic components in accordance with one aspect of the present invention.

Figure 9 is a flow chart showing a method in accordance with one aspect of the present invention.

Figures 10A-10F show side phantom views of various head designs for the device of Figure 1.

Figure 11 shows an exploded side phantom view of the device of Figure 1 fitted with an adapter in accordance with a further aspect of the present invention.

Figure 12 is a block diagram showing electronic components in accordance with an aspect of the present invention where the faceplate does not include an EEPROM.

### DETAILED DESCRIPTION OF THE INVENTION

Disclosed and illustrated generally at 20 in the Figures is a hand held device in accordance with the present invention. The device includes a head 22 and a handle 24. The head 22 of the device includes a faceplate 23 and a baseplate 25. Faceplate 23 comprises an energy source 30, a substrate 57 for supporting energy source 30, and can have an outer surface 26. Substrate 57 and outer surface 26 each define at least one aperture 28 for allowing the distribution of a material 38 from the outer surface 26, as described further below. Substrate 57 can comprise a printed circuit board (PCB) or other structure functionally equivalent thereto. Outer surface 26 is comprised of a generally transparent material which allows maximal transmission of light from energy source 30 to the skin of the user during operation of device 20. Outer surface 26 can be manufactured from such suitable materials as glass, polycarbonate, Macrolon™, and the like. Outer surface 26 of faceplate 23 can further define apertures (not shown) for allowing light from energy sources 30 to be transmitted from outer surface 26. In an embodiment where such apertures are included, the transparency of outer surface 26 is not critical for allowing the transmission of light. Outer surface 26 can also include an energy reflecting surface 27 designed to recycle energy reflected back from the user's skin when the device is in use by reflecting such energy back to the user's skin. The energy reflecting surface 27 can be located between energy source 30 and substrate 57 and can extend beyond the outer edges of energy source 30. Variations of energy reflectind layers may be used as known in the art.

It is also contemplated that device 20 can be used without outer surface 26. In such an embodiment, material 38 can be emitted directly from the at least one aperture 28 of substrate 57 or from at least one suitable extension (not shown) which extends from aperture 28 of substrate 57. In this embodiment, energy source 30 can be suitably protected, if necessary, from exposure to material 38 through means known in the art.

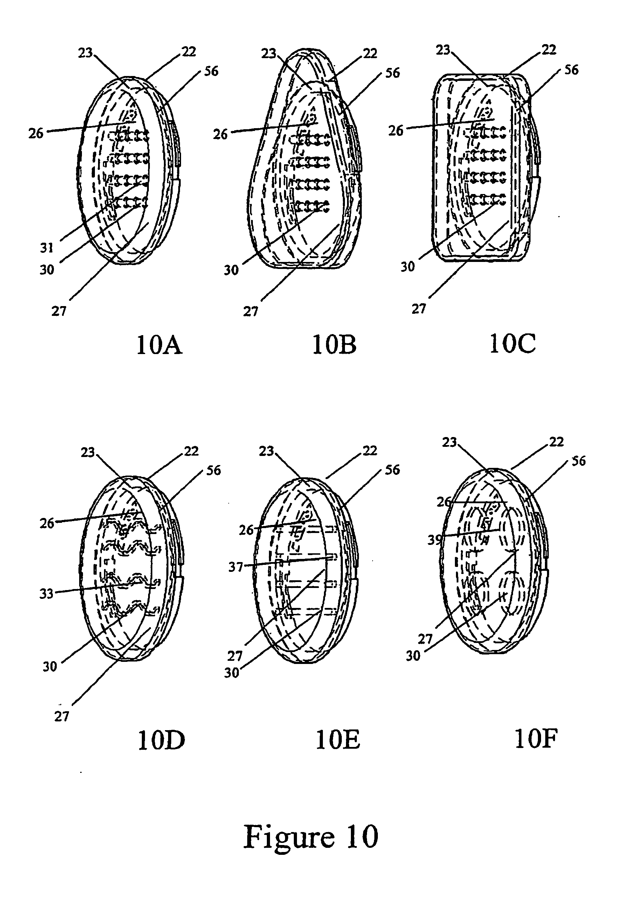

Faceplate 23 of the device can be of varied shape or design, as shown for example in Figures 10A to 10F. The handle 24 is designed to be easy to hold in one hand, and is connected to the head 22 through a flexible neck 68 or a fixed neck (not shown).

The size of the device should be suitable to allow the user to hold it in their hand during use. The faceplate should be of a size that is suitable to treat a portion of a person's skin and tissue.

The energy sources 30 can be of any type and of any wavelength that is suitable for the treatment at hand as known to persons skilled in the art. For example, energy sources 30 with a peak wavelength of about 415 nm and a bandwidth of about 20 nm can be used for the treatment of acne. The preferred embodiment of energy sources 30 for the present invention is one or more light emitting diodes (LEDs), however, the present invention is not limited to the use of these energy sources. Other energy sources including (without limitation) those that deliver microwave energy, radiofrequency energy, ultraviolet, visible, or infrared energy, ultrasound, laser energy, light energy or electrical stimulation, can also be used in place of or in combination with energy sources 30. Examples of known energy sources for delivering such energy include fluorescent lights, sulfur lamps, flash lamps, xenon lamps, LEDs, laser diodes, lasers, and filamentous lights. Examples of head 22 designs having varied energy sources are shown in Figures 10A, 10D, 10E and 10F, namely, a semiconductor energy source 31 such as an LED or a laser diode, a microwave energy source 33, a fluorescent tube 37, and a filamentous energy source 39.

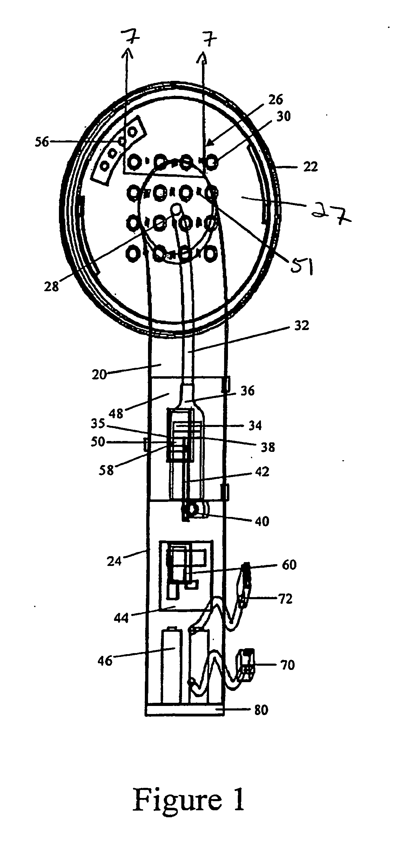

The device 20 utilizes a power source, either internally housed (in the form of a battery 46) or external to the device (through a plug 70 for connecting the device in a standard electrical power receptacle), or both. The battery 46 can be disposable or rechargeable, can consist of one or more cells (for example, 2 cells as shown in Figure 1) and can optionally be accessed for removal from the device by removing battery cover 80.

Housed within the handle 24 is a controller 34, which preferably is activated with a switch 35, through which the user controls the device. The controller 34 allows the user to "tum on" and "turn off" the device, though the turning off of the device can be done automatically by the device at the appropriate end of treatment, as described below. The user can turn on and use the device for a predetermined amount of time based on instructions for a treatment regimen that accompany the device or instructions prescribed by a medical professional. The instructions can be in written, audio, or video form, or can be downloaded from a computing device or computer network. Optionally, the controller 34 can also be used to select a treatment regimen, though this can also be done automatically through the use of coded containers or face plates, as described below.

The handle 24 also houses a processor 44, which can be pre-programmed with suitable treatment regimens. The processor 44 is used to time the duration of treatment, or to pulse or otherwise modify the energy source 30 to optimize the treatment. For example, for the treatment of acne, the user might set the controller 34 to an acne treatment setting (or to an 'on' position if the treatment setting is automated as described below) and then place the device proximal to the skin being treated, and would activate the device through the controller 34. The processor 44 can control or vary the duration, intensity, and pulses of energy being administered to the patient in accordance with the treatment, and can also signal the patient through an audible tone or other method, when the treatment is finished. The processor 44 can also act as a controller for the dispensing of appropriate amounts of material 38 at appropriate times, as further described below.

The head 22 of the device 20 includes a faceplate 23 that can be removed by the user from a baseplate 25 through means such as a bayonet mount 54 or other suitable mounting means, for example, through mechanical fittings. Thus the same device 20 can be fitted with various faceplates 23, each with different energy sources or other operational or structural features. Information can be transferred from the faceplate 23 to the device 20 for use by the processor 44 by a connector 56 disposed on faceplate 23, which is connected to connector 52 disposed on baseplate 25 Connector 52 communicates with processor 44 via cable 86 indicating which faceplate 23 is connected to the device, allowing the processor 44 to identify the treatment regimen that corresponds to the particular faceplate 23. Moreover, in this embodiment, power transmission from device 20 to faceplate 23 and data communication between processor 44 and faceplate 23, when faceplate 23 contains memory, is also accomplished through the connection established between connectors 52 and 56. However, in other embodiments, identification information, power transmission and data communication between any memory present on faceplate 23 and processor 44 can be accomplished using different types of mechanisms and connections. For example, separate pairs of connectors can be used for the transmission of power, communication of identification information and data communication. Alternatively, other connection types can be used for data communications between faceplate 23 and device 20, such as a wireless connection based on radio transmission. Moreover, the identity of a faceplate 23 can be contained on faceplate 23 as an identifier readable by a sensor located on device 20. In yet other embodiments, other methods for identifying the faceplate may be used. An electrical coding can be set into faceplate 23 using alternating bands of conducting and non-conducting material can be read by faceplate sensors located on device 20. Alternate mechanisms of communicating identity information can also be used, such as a bar code and optical sensor system, a magnetic strip and magnetic strip reader, an electrical contact, or a mechanical key recognition system. These and other such variations are within the scope of the invention.

The use of a variety of interchangeable faceplates 23 permits the user to purchase a low cost device that is optimized for the ailment to be presently treated, while allowing the user the flexibility to expand treatment options in the future by purchasing a new faceplate 23, rather than a whole new device. Interchangeable faceplates 23 have the added benefit that they can be easily removed from the device 20 for washing or autoclaving. Alternatively, if costs permit, the device can be manufactured with one form of integrally or permanently attached faceplate only and additional units for other treatments can similarly be manufactured.

A simpler form of therapy device comprising a body, an energy source disposed on the body for emitting a desired wavelength of electromagnetic radiation, preferably light radiation, and a material dispensing system disposed on the body for dispensing a desired material for use with the device, falls within the scope of this invention. This device can operate under manual control, where the user determines the duration and frequency of treatment.

The therapy device can be sold as a kit comprising the assembled device together with instructions for usage and possible treatment regimens or protocols. The instructions can be in written, audio, or video form, or can be downloaded from a computing device or computer network. The instructions can be provided by a medical professional. The therapy device can be sold unassembled as part of a kit, in which case the kit can further comprise instructions for assembly of the device.

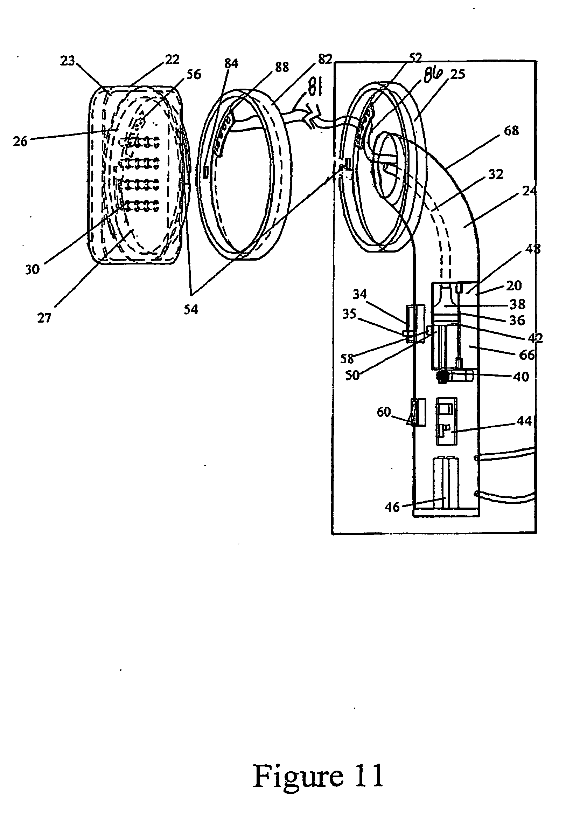

The device can also include an adapter 82 for receiving faceplate 23 and facilitating the use of the device over treatments when it can become uncomfortable to hold the entire device with the handle over time. The adapter 82, shown fitted to the device in Figure 11, contains faceplate attachment means 84, which are compatible with and complementary to the bayonnet mount 54, and an extension cable 81 running from the faceplate attachment means to the baseplate 25 and functionally connected thereto. Extension cable 81 would allow the powering of the energy source 30 on faceplate 23 by the battery 46 and processor 44 on the handle 24 through the extension cable 86. The adapter 82 can also contain adapter connector 88 for the remitting of faceplate information to processor 44, also through the extension cable 86 which can be connected to the connector 52 or the baseplate 25 to facilitate such remitting of information. The adaptor 82 can also contain straps for affixing faceplate 23 (connected to adaptor 82) to the user during treatment (not shown).

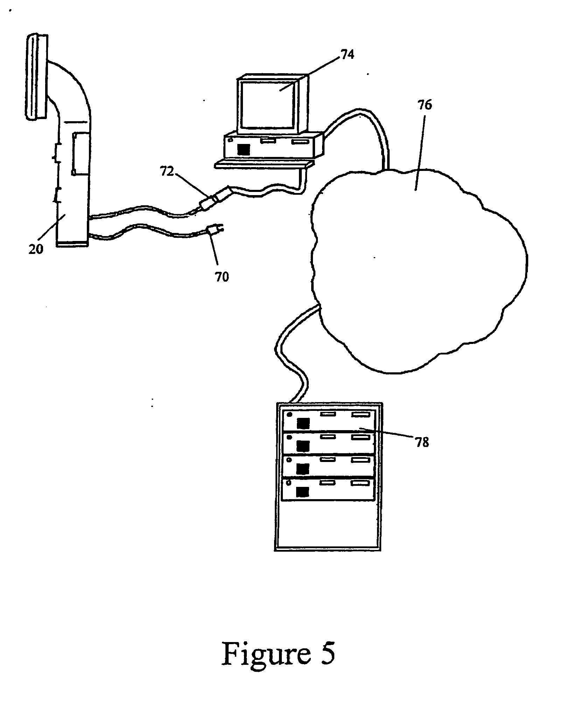

The device 20 can also provide for updating the software of the processor 44 as new faceplates 23 or new treatment regimens are designed. Referring to Figure 5, processor 44 can connect by wire connection through firewire 70 or by Universal Serial Bus 72, as shown, or through another wired or wireless communication means such as an Infrared port (not shown) to a personal computer 74 connected through the Internet 76 to a database server 78 containing an updated database of treatment regimens. Such communication can also occur through a wireless local area network, bluetooth, or other communications technology (not shown) or through the insertion of a flash card or other memory-containing device which contains pre-programmed instructions or data (not shown). Updating of the device can be passive (occurring in real-time as new software is developed) or active, occurring only at the request or command of the user. Alternatively, the device can be pre-programmed with treatment protocols and can not have software updating means.

Although faceplate 23 of device 20 can be interchanged with other faceplates 23 to optimize the treatment regimen for a variety of ailments, it is possible that a single faceplate 23 would contain a suitable energy source 30 for a broad subset of ailments. For example, faceplate 23 can include an energy source comprised of LEDs for emitting electromagnetic radiation at about 410 nm and other LEDs for emitting electromagnetic radiation at about 630 nm. Other medically useful electromagnetic emissions occur at about, for example, 580 nm, 660 nm, 680 nm, 800 nm, 810 nm, 820 nm, 830 nm, 840 nm, and 900 nm, with a band width of about 40 nm, more preferably about 20 nm. Therefore, depending on the flexibility of head design, it can be necessary to have a secondary way in which to select treatment regimens. For example, the controller 34 can include a user interface (not shown) to allow the user to program or select various treatment regimens by hand. Optionally, an automated mechanism for the determination of treatment regimens is possible, such as by using coded, treatment-specific containers, as described below.

The device preferably also includes a system for dispensing a desired material 38 such as a gel or lotion for use in the treatment. During treatment using device 20, material 38 is disposed between the skin of the user and faceplate 23. Material 38 can optimize and/or enhance the energy transfer between energy source 30 and the skin of the user by filling in irregular voids that exist on the surface of the skin. Another function of material 38 can be to alter the refractive index of the skin or target tissue so that the absorption spectrum of the skin or target tissue is closer to the emissions spectrum of the source of electromagnetic radiation. This aspect of material 38 can be useful because the skin has an index of refraction of about 1.4 in the visible and the near infrared, which is larger than that of air. As a result, any photon that interacts with the **air-**skin interface is deflected if it does not hit the skin at an incidence angle of substantially 0°. Since the surface of the skin is irregular, the angular distribution of the skin increases. In order to enhance the absorption of light into the skin, material 38 can comprise components that have an index of refraction which is close to that of skin. Such components are sometimes called skin index matching materials. An example of a suitable index matching material is propylene glycol solution with a refractive index of 1.5. Material 38 can thereby enhance the absorption by the user's skin of photons emitted by energy source 30 by improving the surface irregularities of the skin and minimizing the difference of the indices of refraction between the skin and the area between the skin and the faceplate 23. Material 38 can also act as a lubricant or hydration agent that provides a low friction surface coating for improving the comfort and operation of the device. For example, material 38 can comprise a gel, such as a water based gel. Material 38 should be transparent to the beneficial light emitted by energy source 30. In a preferred embodiment of the present invention, outer surface 26 of device 20 is pressed or placed against with the surface of the user's skin thereby causing the surface of the skin to be substantially contiguous with outer surface 26, and material 38 is disposed therebetween during treatment with device 20.

Material 38 can also contain a medicament, active ingredient, or supplement known to be useful in treatment of a specific indication. For example, the material 38 can contain an acne treatment such as benzoyl peroxide, preferably in a concentration of about 0.1% to 10%. This allows the user to combine a treatment using electromagnetic radiation with a more conventional treatment for their ailment using minimal steps. The material can also contain ingredients such as aloe, Vitamin E, a hydration agent, Vitamin C, Vitamin D, Vitamin A, Vitamin K, Vitamin F, Retin A (Tretinoin), Adapalene, Retinol, Hydroquinone, Kojic acid, a growth factor, echinacea, lanolin, an antibiotic, an antifungal, an antiviral, neutraceuticals, cosmeceuticals, a bleaching agent, an alpha hydroxy acid, a beta hydroxy acid, salicylic acid, antioxidant triad compound, a seaweed derivative, a salt water derivative, algae, an antioxidant, a phytoanthocyanin, phthalocyanine, a phytonutrient, plankton, a botanical product, a herbaceous product, a hormone, an enzyme, a mineral, a genetically engineered substance, a cofactor, a catalyst, an antiaging substance, insulin, trace elements (including ionic calcium, magnesium, etc.), minerals, minoxidil, a dye, a natural or synthetic melanin, a metalloproteinase inhibitor, proline, hydroxyproline, an anesthetic substance, benzoyl peroxide, amino levulinic acid, chlorophyll, bacteriachlorophyll, Coenzyme Q10, copper chlorophyllin, chloroplasts, carotenoids, phycobilin, rhodopsin, anthocyanin, and derivatives, subcomponents, immunological complexes and antibodies directed towards any component of the target skin structure or apparatus, and analogs of the above items both synthetic and natural, as well as combinations thereof. It will be noted by those skilled in the art that the medicaments, active ingredients, and/or supplements disclosed herein and their equivalents, as well as any other medicaments, active ingredients, and/or supplements that can be useful when used in combination with the device and methods of the present invention, can also have index matching and skin smoothing properties that can contribute to the effectiveness of the treatment.

The medicament, active ingredient, or supplement can be photosensitive and can undergo a photochemical reaction when applied to the skin and exposed to energy source 30.

The medicament, active ingredient, or supplement can be administered to the skin or target tissue before use and separately from administration of material 38 and use of device 20.

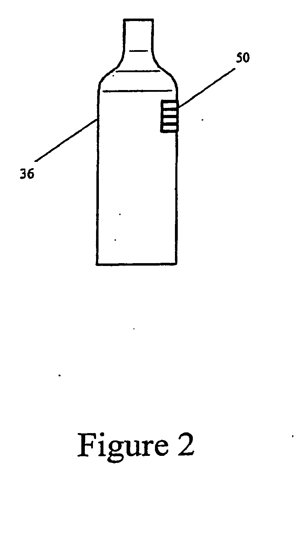

A removable container 36, shown in isolation in Figure 2, containing material 38, as described above, is housed within a container receiver 48, such as a cavity or slot within the device, preferably located on the device handle and preferably having a door 66 that is retractable or that is rotatably mounted on hinges 64 and lockable in a closed position using latch 62, or a similarly resealable opening for insertion and removal of the container. The container can be disposable, or can be refillable. The container can also be filled by a dermatologist, pharmacist, or other medical professional, for prescription dispensing or customizable formulations of material.

The material 38 is squeezed out of the container either manually by the user using, for example, a spring-loaded trigger mechanism 60 operably connected to a plunger 42 or other squeezing mechanism or pump mechanism for displacing material 38 out of the container 36, or automatically and under processor 44 control when the device is activated, using, for example, an electric motor 40 or solenoid (not shown) operably connected to a plunger 42 or other squeezing mechanism for displacing material 38 out of the container 36, and a motor control system for turning on and off the motor (as shown, incorporated within processor 44). As would be recognized by those skilled in the art, other suitable pumping or dispensing mechanisms could be used, such as a diaphragm pump, which would also aid in dispensing a predetermined amount of material 38 during operation of device 20.

The material 38 displaced out of the container 36 is forced through one or more apertures 28 on the device head 22, either directly (not shown) or through one or more distribution passages 32 built into the device and connecting the container 36 to the apertures 28. In this manner, the appropriate amount of material 38 is distributed directly to the space between the skin of the patient (not shown) and faceplate 23 with little effort from the user.

In order to make the therapy easy to administer, the container 36 can be clearly labelled for the ailment it is meant to treat, as well as with its ingredients. Various materials can therefore be prepared and sold separately, in containers compatible with the device, for repeat applications of a wide variety of treatments for a wide variety of ailments.

In order to simplify the use of the device, individual containers 36 can be designed to communicate with the device. For example, an electrical coding 50 set into the container 36 using alternating bands of conducting and non-conducting material can be read by container coding sensors 58 on the device 20. Alternate communication means can also be used, such as a bar code and optical sensor system (not shown), a magnetic strip and magnetic strip reader (not shown), an electrical contact (not shown), or a mechanical key recognition system (not shown).

In this manner, the device can determine which treatment is to be performed based on which container 36 is in the device. The processor 44 can determine the appropriate rate and amount of material 38 to be displaced from the device 20 during treatment, based on the type of material 38 in the device as read by the sensors 58 from the data on the container's electrical coding 50. In this way, the processor 44 acts as a form of "motor control system" for the dispensing of the material.

The processor 44 can also determine whether the appropriate faceplate 23 is on the device for the treatment required (based on the type of material that is loaded in the device), and can activate the energy sources 30 for the appropriate treatment regimen (again, based on the type of material 38 that is loaded in the device 20). For example, when an "acne" material 38 is loaded into the device 20, the processor 44 "reads" the type of material loaded using container coding sensors 58, accesses its internal database to determine what the appropriate treatment regimen is for acne (including type, duration and intensity of energy emission, (for example, an 18 minute treatment with combination 620 nm and 415 nm LEDs with a total energy output of 40-90 joules (J) per session in a continuous wave)) as well as what is the appropriate timing, rate, and amount of material to be dispensed.

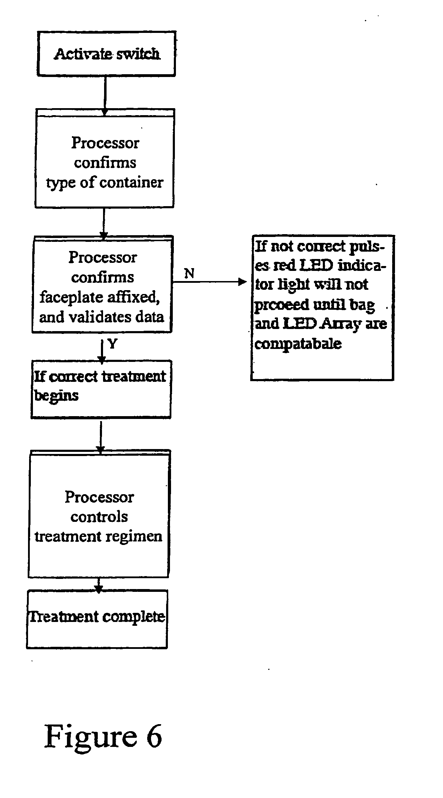

A flow chart showing generally an example of how the processor 44 can process information from the container 36 and the faceplate 23 is shown in Figure 6.

The processor 44 verifies that the correct faceplate 23 is on the device by communicating with faceplate 23 through connectors 52 and 56 (to ensure, for example, that a faceplate 23 with light sources 30 capable of emitting light at 415 nm is affixed to the device). The device will activate when the controller 34 is deployed, and when the appropriate faceplate 23 and container 36 are affixed to the device. Optionally, the device can also use a counter, sensors or other means, typically affixed to the plunger 42, to determine whether there is enough material 38 in the container 36 to undergo the appropriate treatment regimen, and will warn the user if the amount of material is insufficient, or if the container is nearly empty.

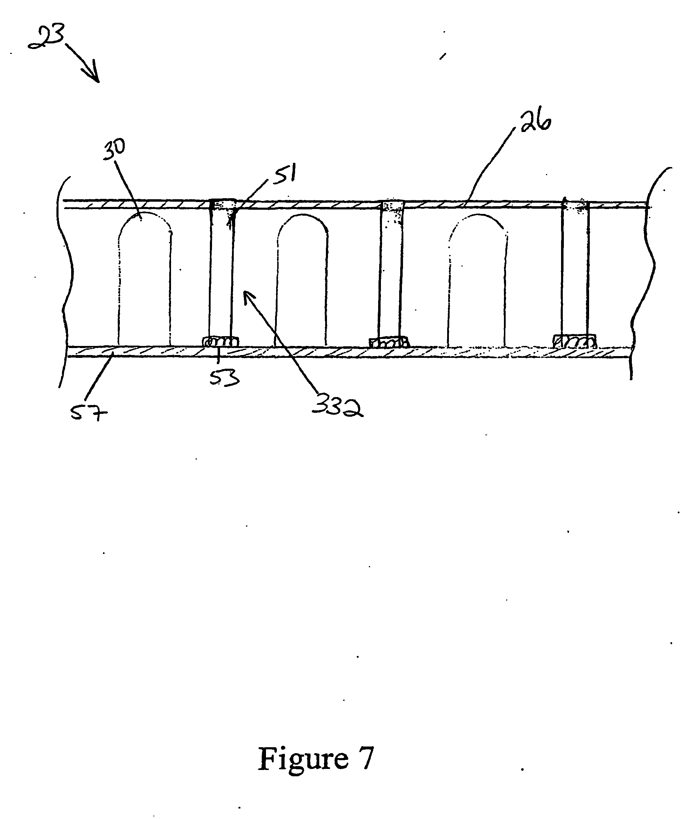

Optionally, device 20 can further comprise a temperature sensing device such as temperature sensing device 332 shown, for example, in Figure 7, for measuring the temperature and/or any change in temperature at the skin-device interface. Temperature sensing device 332 comprises a thermal conductive material 51, such as copper metal, and a thermal transducer 53, such as a thermosistor. Thermal conductive material 51 extends from outer surface 26 and is in communication with thermal transducer 53. Thermal transducer 53 is in communication with processor 44. It will be understood by those with skill in the art that other devices which incorporate the functions of temperature sensing device 332 would be suitable. For example, a temperature sensing device which is flush with at least part of the face plate that contacts the user's face during use is contemplated.

It will be recognized by those with skill in the art that the configuration of energy source 30, and temperature sensing device 332 (if used) can be implemented on a PCB or other structures functionally equivalent thereto.

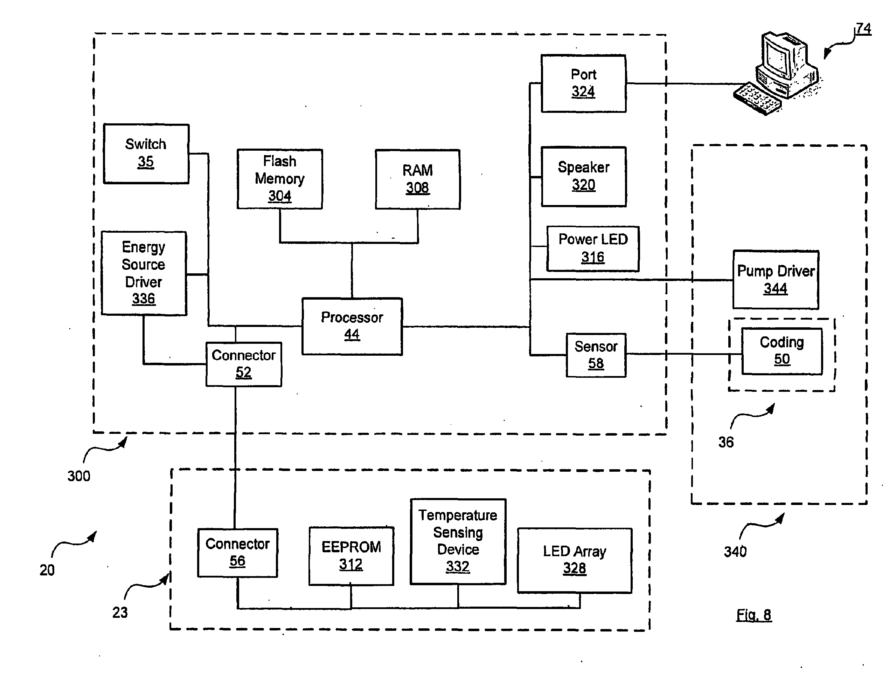

Referring now to Figure 8, a block diagram of certain components of device 20 are shown according to an embodiment of the invention. In this embodiment, device 20 includes a control mechanism 300, a faceplate 23 and a dispensing system 340.

Control mechanism 300 has a processor 44. Processor 44 is connected to a persistent storage device which, in this embodiment, is a flash memory 304 containing a plurality of applications executable by processor 44, and related data that enables device 20 to perform certain functions. Processor 44 is also connected to a random access memory unit ("RAM") 308. Processor 44 can send output signals to various output devices including alarm sources which in this embodiment are an LED 316, and a speaker 320. Processor 44 can also receive input from various input devices including switch 35 and sensor 58.

Control mechanism 300 also includes an energy source driver 336. Energy source driver 336 is operable, through a control signal from processor 44, to deliver a driving current to the energy source located on faceplate 23. Both processor 44 and driver 336 are also connected to connector 52. In this embodiment, connector 52 is a five pin surface mount connector.

Control mechanism 300 is operable to communicate with faceplate 23 through connector 52. Faceplate 23 includes an energy source, which in this embodiment is an LED array 328, an electrically erasable programmable read only memory (EEPROM) 312 and a temperature sensing device 332, all of which are in communication with control mechanism 300 through connector 56 which connects to connector 52.

In a preferred embodiment, LED array 328 contains thirty six LEDs, each LED capable of generating about 12mW of power at about 100% duty cycle under continuous operation at a current of about 25mA, which current is supplied by energy source driver 336 therough connectors 52 and 56. In other embodiments, other types of LEDs with different operational characteristic can be used and these embodiments are within the scope of the invention.

EEPROM 312, in this embodiment, is a 1-wire EEPROM as, for example, manufactured by Maxim Integrated Products, Inc.of California U.S.A. Temperature sensing device 332, in this embodiment, is a solid state temperature sensing device such as MCP9700, a low-power voltage output temperature sensor, manufactured by Microchip Technology Incorporated of Chandler AZ, U.S.A.

EEPROM 312 is responsible for storing additional data relevant to the performance of certain functions. This data is accessible by processor 44 through connector 52. As now apparent to those skilled in the art, in other embodiments, other persistent storage devices such as a ROM or flash-memory can be used in place of an EEPROM for storing the additional data on face place 23 and these embodiments are within the scope of the invention. Temperature sensing device 332 detects the temperature of faceplate 23 and is operable to convey this temperature reading to processor 44. LED array 328 is operable to deliver an energy according to a current supplied by energy source driver 336.

Control mechanism 300 is also operable to communicate with dispensing system 340. Dispensing system 340 includes a pump driver 344 and a coding 50 which is located on container 36. Driver 344 is operable, through a control signal from control mechanism 300, to drive a solenoid to deliver a predetermined amount of fluid contained within container 36. In this embodiment, container 36 is a container for delivering benzoyl peroxide with a concentration of 1 % which is delivered from container 36 to the skin by a series of pumps or pulses actuated by driver 344. Coding 50, as previously described, is formed from alternating bands of conducting and non-conducting material. The coding combination allows a different voltage to be returned to processor 44 corresponding to different container types. As it is now apparent to those skilled in the art, in other embodiments, other coding mechanisms can be used such as different resistors

Processor 44 is also operable to communicate with a computing device 74 through an interface operable to conduct communications when a computing device 74 is optionally connected to the interface. In this embodiment, the interface is the communications port 324 which uses communications protocol Ras-232 known to those skilled in the art, and hence is a serial port. As it is now apparent to those skilled in the art, in other embodiments, other types of communication interfaces can be used for connecting to a computer. These interfaces include but are not limited to Universal Serial Bus (USB), infrared (IR), Blue Tooth, two-way radio, wired Ethernet and wireless Ethernet connection using a variety of protocols such as 801.11g or 801.11b. Moreover, the type of computing device that can be connected to device 20 includes, but is not limited to, a desktop personal computer (PC), a laptop, a personal digital assistant (PDA) or any other mobile or stationary device that is capable of communicating, processing and storing information.

Control mechanism 300 maintains a treatment database 200, used for determining different parameters of a treatment regimen. Database 200 contains information relevant to treatment regimens such as the duration of a specific treatment and duration and intensity of energy delivered during a cycle. Accordingly, a separate database record exists for each different treatment regimen. Typically, records are stored in database 200, which is maintained in flash-memory 304. However, some records, or portions thereof, can also be stored in EEPROM 312 located in faceplate 23. The records or portions thereof that are maintained in EEPROM 312 contain information that is specific to the treatment regimen or regimens that are deliverable using that particular faceplate 23. Table I shows an example record 204, labeled Record #1 that contains data for an example acne treatment regimen and is maintained in EEPROM 312.

**Table I**

| **Example record 204 for an example acne treatment regimen** | | |
|---|---|---|
| **Record #1** | | |
| | **Field Type** | **Value** |
| **Field 1** | **Treatment type** | Acne |
| **Field 2** | **Initial Duration of each cycle** | 90 seconds |
| **Field 3** | **LED Efficiency** | 12mW/25mA |
| **Field 4** | **Form of Activation** | 40 |
| **Field 5** | **Target power delivery per cycle** | 30 J |
| **Field 6** | **Upper heat limit** | 41° C |
| **Field 7** | **Lower heat limit** | 35° C |
| **Field 8** | **Number of pump pulses** | 3 |
| **Field 9** | **Container type** | 5 |
| **Field 10** | **Energy Source** | LED array |

Describing Table I in greater detail, Field 1 contains the type of treatment regimen contained in this record which in this example is a regimen for Acne. Field 2 contains the initial duration of each treatment cycle, while Field 3 contains the operating efficiency of LEDs found in LED array 328. These fields are set to about ninety seconds and about 12/25 mW/mA respectively for this example regimen. Field 4 contains the form of activation for LED array 328. In this embodiment, two forms of activation are possible. The first is continuous wave where LED array 328 is activated continuously, and the second is pulse wave, where LED array 328 is activated in pulses. In this example, Field 4 is set to 40 meaning that during a cycle, LED array 328 is to be activated at a pulse rate of about forty Hertz.

Activating LED array 328 at a certain current for a specified period of time results in a certain amount of irradiance power being delivered by LED array 328. Accordingly, the irradiance power to be delivered during a cycle is the amount of power generated corresponding to the time, current and form of activation of LED array 328 specified in Fields 2 through 4 and is based on the efficiency of LEDs used (which is specified in Field 3). This power, referred to as target cycle power, is specified in Field 5. In this example, it is targeted that a combination of LEDs used should result in the delivery of about thirty Joules by LED array 328 when LED array 328 is activated at about a forty Hz pulse for a ninety second period. Accordingly, in this example, Field 5 is set to about thirty Joules.

Continuing with the description of Table I, Fields 6 and 7 specify temperature limits within which device 20 is to keep the temperature of faceplate 23 during the treatment. In this example, the temperature is maintained between about thirty-five and about forty-one degrees centigrade. Field 8, contains the number of times container 36 is to be pumped prior to the activation of LED array 328. This field, in effect, determines the amount of fluid to be delivered by container 36 during a treatment. In this example, container 36 is to be pumped 3 times. Field 9 specifies the type of container this regimen will work. In this case, the container with a code of 5 is the appropriate container to be used. In other embodiments, multiple container types can be deemed compatible with a treatment regimen and such embodiments are within the scope of the invention. Field 10 specifies the type of energy source present on the corresponding faceplate. In this example an LED array is used.

control mechanism 300 also maintains several variables such as a power down timer 208 used for counting down to the point where device 20 is to enter a low power mode, in effect shutting it down. A treatment timer 212 and a power counter 216 are also maintained to track the amount of time and power that has been delivered in a given cycle. Control mechanism 300 also maintains logging data which contains information about the usage of device 20, that can be used in making determinations about the efficacy of the treatments used and the maintenance of device 20. For example, logging data can comprise logging variables that include counters for counting the duration that LED array 328 has been activated, causing an alarm to be delivered if LED array 328 have been used for ninety percent of their useful life, reminding the user to replace the faceplate. In another example, a doctor could monitor a patient's use of the device by examining the logging variable that tracks how long device 20 has been used since a particular date. In this embodiment logging data are maintained in flash memory 304. In other embodiments, logging data could be maintained in a separate storage device dedicated to storing these variables such as an additional flash-memory unit or an EEMPROM. These and other variations are within the scope of the invention.

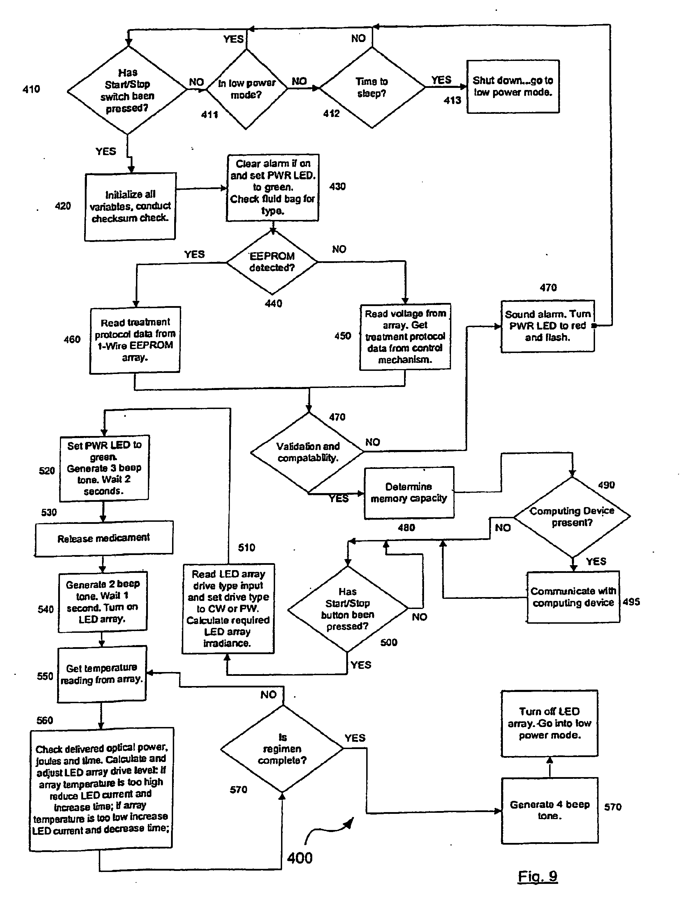

Referring to Figure 9, a method for delivering a treatment regimen is indicated generally at 400. In order to assist in the explanation of the method, it will be assumed that method 400 is performed using device 20. Furthermore, the following discussion of method 400 will lead to a further understanding of device 20 and its various components. (However, it is to be understood that device 20 and/or method 400 can be varied, and need not work exactly as discussed herein in conjunction with each other, and that such variations are within the scope of the invention).

The current performance of method 400 is initiated by pressing switch 35 to turn the device on while device 20 is in a low power mode. Referring to Figure 9, at step 410, a determination is made whether switch 35 has been pressed. While in low power mode, device 20 loops through step 410 continuously. Hence, when switch 35 is pressed, the determination is made that switch 35 was pressed, and, accordingly, method 400 advances to step 420.

At step 420, device 20 is initialized. In this example, variables maintained by device 20 are set up in RAM 308 and initialized. For example, timer variable 208 that counts down to a time when device 20 is to enter a low power mode is set to 300, meaning that 300 seconds remains to power down. The value of power down timer 208 is decremented in the background throughout the performance of method 400 Treatment timer 212 and power counter 216 are set to zero since the treatment has not started at this point. In addition, logging variables are also moved to RAM 308 and initialized with the values currently stored in flash memory 304 so that they can be updated, as appropriate, during this activation of device 20. Moreover, a checksum of flash memory 304 is performed in the usual manner to check for proper operation.

Moving to step 430, the type of container 36 present in device 20 is determined. In this embodiment, step 430 is performed by having processor 44 read the electrical coding 50 set into container 36 through sensors 58. In this example, it will be assumed that container 36 is filled with benzoyl peroxide with a concentration of 5% and that the container code is 5. Also, at this step, power LED 316 is set to green indicating normal operation of the unit. Furthermore, any output to speaker 320 is discontinued to turn off any ongoing alarms.

Continuing with the performance of method 400, at step 440, a determination is made as to the type of faceplate 23 that is present on device 20. This is accomplished by having processor 44 detect the presence of an EEPROM, through connector 52, using a presence pulse or other suitable known methods. In this example, as discussed above, it is assumed that faceplate 23 does contain an EEPROM 312. Accordingly, step 460 is performed.

At step 460, treatment regimen data is retrieved. In this example, record 204 is retrieved by processor 44, from EEPROM 312. Specifically, processor 44 moves the record 204 to RAM 308 for use during the delivery of the treatment. In other embodiments where faceplate 23 can be used with more than one treatment regimen, the user can be presented with a selection of regimens available and asked to pick the one to be used. Subsequently, the record associated with the selected regimen would be retrieved. For example, the selection can be made by pressing switch 35 a prescribed number of times. These and other such embodiments are within the scope of the invention.

At step 470, the contents of record 204 are validated. In this embodiment, the validation is performed by ensuring that the fields of record 204 are not blank. In other embodiments, other methods of data validation can be used. For example, a separate database could be maintained in ROM 304 or EEPROM 312 specifying valid ranges of values for the fields of a record. Each record can then be validated against this second database. In this example, it is assumed that the record 204 contains valid data. As part of the validation step, the type of container detected at step 430 is compared to Field 9 of record 204 which specifies compatible containers. In this example, Field 9 has a value of five which is the container code detected at step 430. Accordingly, container 36 is deemed compatible with faceplate 23.

Continuing with the performance of method 400, at step 480, the capacity of RAM 308 is determined. If RAM 308 is full, namely no free memory remains to be used during the delivery of the treatment regimen, processor 44 generates an alarm by sending a signal to power LED 316 causing it to turn yellow and flash. In this example, it is assumed that there is memory remaining for use, and hence, no alarm is generated. As it is now apparent to those skilled in the art, different criteria can be used in determining the capacity of RAM 304. For example, in other embodiments, an alarm can be generated if greater than a certain percentage, such as greater than ninety five percent, of RAM 304 is occupied. These and other variations are within the scope of the invention.

At step 490, the presence of a connection to a local computer is detected. In this embodiment, processor 44 determines, in the usual manner, whether a computing device 74 is attached to the RS-232 port of device 20. In the present embodiment it is assumed that a device is attached. Accordingly, step 495 is performed.

At step 495, logging data are uploaded to computing device 74, freeing the corresponding space in flash memory 304. In this example, processor 44 removes the logging data from flash memory 304, and transfers it to computing device 74 through port 324. In other embodiments, other events in addition to the detection of computing device can be added to initiate uploading of logging data to computing device 74. For example, a user at computing device 74 can be asked to initiate uploading by providing a command such as a mouse click, at computing device 74. Alternatively, device 20 can deliver an alarm to the user when the presence of computing device 74 is detected and wait for a response from the user in the form of pressing switch 35. These and other such embodiments are within the scope of the invention.

At step 500 a determination is made whether switch 35 has been pressed. Pressing switch 35 allows the treatment to begin. Otherwise, method 400 loops through step 500 until switch 35 is pressed. In this example, it is assumed that switch 35 is pressed, indicating that the treatment is to begin.

Continuing with method 400, at step 510, the properties of irradiance to be delivered is determined. To determine the properties of irradiance, the type of energy source on faceplate 23 is determined. In this embodiment, the type of energy source on faceplate 23 is an LED array 328, as discussed above. Processor 44 determines the presence of LED array 328 from Field 10 of record 204. Moreover, based on Field 8 of record 204, processor 44 sets the form of activation for LED array 328 to be either in the form of a pulse wave (PW) or a continuous wave (CW). In this example, a pulse wave at a rate of forty Hertz is used. In other embodiments other methods could be used for detecting the type of energy source and the form of activation. For example, control mechanism 300 can check the voltage across the connection to the energy source and make a determination based on the voltage value read. In yet other embodiments, the user can manually specify the activation form. These and other similar embodiments are within the scope of the invention.

Continuing with step 510, using record 204, target irradiance power, initial operating period and LED efficiency for LED 328 are obtained from Fields 5, 2 and 3 respectively of record 204. In this example target irradiance power for a cycle is about thirty J, the initial operating period is about ninety seconds, and the LED efficiency is about 12mW/25mA. Based on these values, the initial operating current is calculated. In this example, the initial operating current is chosen such that LED array 328 can deliver about thirty mJ in about ninety seconds. It is assumed that in this case, the initial operating current is calculated to be about 25mA.

Having determined the properties of irradiance, at step 520 the user is warned that container 36 is to be activated. In this example, processor 44 delivers signals to power LED 316 and speaker 320 changing the color of power LED 316 to green and sounding two long beep tones. Processor 44 then causes a two second delay before continuing with method 400. As it is now apparent to those skilled in the art, in other embodiments, user warnings can be varied according to a number of criteria such as the amount of attention that needs to be drawn to the activation of pump 36, and the time necessary to prep the start of the treatment from the time switch 35 is pressed.

Continuing with method 400, container 36 is activated. Control mechanism 300 activates container 36 by sending a signal to pump driver 344, which causes a certain amount of material 38 contained in container 36 to be pumped out. The number of activations or pulses is determined according to Field 8 of record 204. In this example, activation is for three pulses in accordance with the example record shown in Table I. In other embodiments, container 36 can be activated manually. For example, device 20 can generate a long beep for each manual activation to enable a user to release the correct amount of medicament manually. These and other such embodiments are within the scope of the invention

At step 540 the user is warned that energy source 30 is to be activated. In this example, processor 44 delivers a signal to speaker 320 sounding a three long beep tones. Processor 44 then causes a two second delay before proceeding with method 400. As it is apparent to those skilled in the art, in other embodiments, user warnings can be varied according to a number of criteria such as the amount of attention that needs to be drawn to the start of the treatment, and the time necessary to prep the start of the treatment from the time switch 35 is pressed. Following the two second delay, the energy source is activated, signifying the start of the treatment cycle. In this embodiment, LED array 328 is activated by a driver current originating from energy source driver 336 of control mechanism 300. Moreover, cycle timer 212 is initialized to a value of zero.

At step 550 temperature reading is obtained from faceplate 23. In this example, processor 44 obtains a temperature reading from temperature sensing device 332. It is assumed that the reading is about forty one and a half degrees centigrade.

At step 560, cycle timer 212 is updated to reflect the amount of time that LED array 328 has been active so far during this cycle. Moreover, the power delivered so far in the cycle is updated by updating power counter 216. Also at step 560, a determination is made whether the temperature is within limits. In this example, the temperature reading obtained is compared to the upper and lower limits specified in fields 6 and 7 of record 204. Accordingly, a determination is made that the temperature is not within limits, and the drive level for LED array 328 is adjusted. Specifically, in this example, array drive current and the duration of the cycle are adjusted. If the temperature read is too high, as it is in this case, LED array 328 drive current is reduced and cycle time is increased. If the temperature read is too low, LED array 328 drive current is increased and cycle time is decreased. As it is now apparent to those skilled in the art, these changes are done to maintain substantially the same target power delivery during a cycle while maintaining temperatures within specified limits. For example, where the temperature is too high, as it is in this example, reducing the drive current to LED array 328 reduces the heat output, allowing the heat to dissipate more readily. However, since the cycle time is increased, overall power delivered during the cycle can remain the same.

Continuing with method 400, at step 570, a determination is made as to whether the regimen is completed. In this example, the value of timer variable 212 is compared to the duration of cycles specified in Field 3 of record 204. If the cycle timer value is less, then the process is not complete, and method 400 loops back to step 550. In other embodiments other methods of determining the completion of the regimen can be used. For example, the power delivery can be used as a basis of determining a regimen's completion, deeming a regimen complete only if the target power has been delivered according to power counter 216. This and other such embodiments are within the scope of the invention. At this point in this example, it will be assumed that the cycle is not complete, causing step 550 to be performed again.

Continuing with the performance of method 400, after several performances of the loop that starts at step 550, a determination is made that the cycle is complete. Accordingly, step 580 is performed. At this step, the user is warned of the cycle's completion by sounding a four beep alarm through speaker 320. At step 590, the treatment is terminated. LED array 328 is turned off by cutting off its driving current. Device 20 is powered down and enters the low power mode where it awaits switch 35 to be pressed, at which point method 400 is performed again starting at step 410.

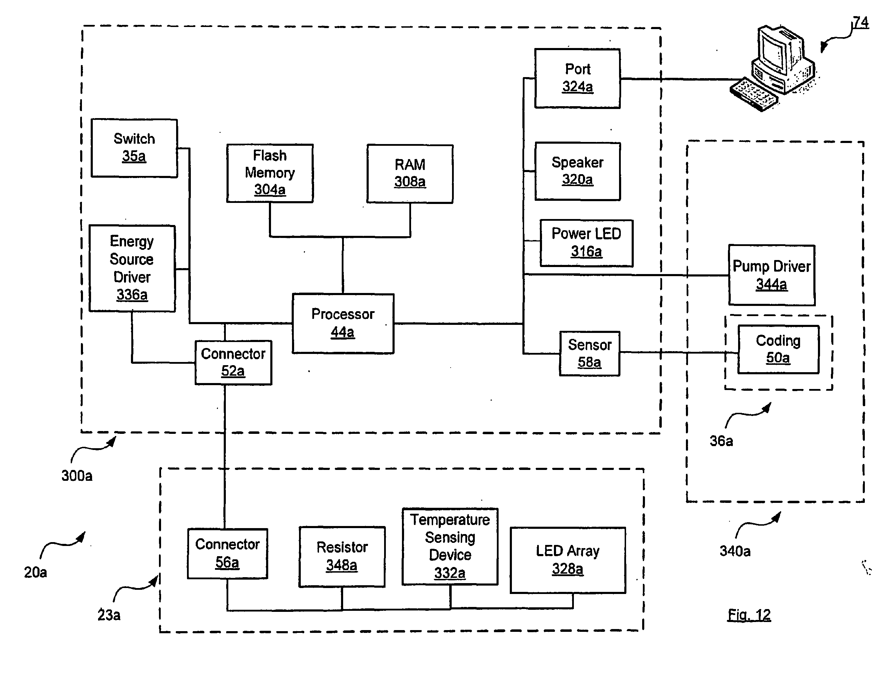

Performing method 400 using different embodiments of device 20 can result in performances that proceed differently than the example performance discussed above. For example, as shown in Figure 12, method 400 can be performed by a device 20a to which a different faceplate, faceplate 23a that does not include an EEPROM 312. In this case, a resistor 348a is present on faceplate 23 to identify the type of faceplate. Device 20a is otherwise substantially the same as device 20 except that the reference numbers of components of device 20a include the suffix "a". Performance of method 400 using device 20a leads to several variations from the first example performance. One variation is that, at step 440, an EEPROM will not be detected, causing step 450 to be performed in place of step 460. At step 450, the type of faceplate is detected. The electrical coding used is in the form of a different voltage corresponding to different resistors used for faceplate types. Accordingly, data corresponding to record 204a is retrieved, by processor 44, in accordance with the faceplate type detected at step 450. Moreover, the record is retrieved from database 200a rather than from an EEPROM since there is no EEPROM present on faceplate 23a. The retrieved data is moved to RAM 308a for use during the delivery of the treatment.

Performing method 400 using device 20 which is at different operational states can also result in performances of method 400 that proceed differently than the example performance discussed above. For example, data contained in database 200 can be corrupted. Accordingly, at step 470 the data is determined to be invalid. Thus, step 475 is performed and an auditory alarm is sent to speaker 320, while power led 316 is signaled to turn red and flash. Following the alarm, method 400 advances to step 410 to determine whether the user has responded to the alarm by pressing switch 35 (for example, in response to the alarm, the user can reattach faceplate 23 to correct problems originating from improperly attached faceplate, or swap faceplates to correct problems originating from faulty faceplates). If so, method 400 is performed again, going through the same initialization and detection steps that brought method 400 to the validation step during the initial performance of method 400 that generated the alarm. If switch 35 is not pressed, processor 44 determines whether it is in low power mode at step 411. If so, it loops back to step 411. Otherwise, processor 44 determines whether it is time to enter a low power mode by first retrieving the value of timer variable 204 determining whether it has reached a value of zero at step 412. If it is determine that it is time to enter low power mode, device 20 enters the low power mode at step 413 until switch 35 is pressed . Otherwise, method 400 loops through steps 410, 411, and 412 until it is time to enter the low power mode or until switch 35 is pressed.

The device, method, and the material described herein can be used in combination with a medicament, active ingredient, or supplement.

For example, an acne treatment or prevention regimen using benzoyl peroxide and/or salicylic acid can comprise the application of a composition comprising benzoyl peroxide and/or salicylic acid to the affected area twice daily. The composition can comprise from about 0.5% to about 10% of benzoyl peroxide and/or salicylic acid, more preferably from about 0.8% to about 7% of benzoyl peroxide and/or salicylic acid, and even more preferably from about 1.0% to about 6.5% of benzoyl peroxide and/or salicylic acid by weight or volume is applied to the area of skin to be treated. The treatment can comprise starting with a composition comprising about 5% of benzoyl peroxide and/or salicylic acid and decreasing the dose in subsequent treatments to about 1 or 2 % benzoyl peroxide and/or salicylic acid. Material 38 is then applied to the skin, and the skin is subsequently exposed to an energy source for a duration of time. Alternatively, material 38 can be applied to the skin before the composition.

According to another example of a method for treating or preventing acne using the present invention, material 38 itself comprises benzoyl peroxide and/or salicylic acid. Material 38 can comprise from about 0.5% to about 10% of benzoyl peroxide and/or salicylic acid, more preferably from about 0.8% to about 7% of benzoyl peroxide and/or salicylic acid, and even more preferably from about 1.0% to about 6.5% of benzoyl peroxide and/or salicylic acid by weight or volume. The treatment can comprise starting with material 38 comprising about 5% of benzoyl peroxide and/or salicylic acid and decreasing the dose in subsequent treatments to about 1 or 2 % benzoyl peroxide and/or salicylic acid. The skin is subsequently exposed to the therapy device for a desired duration of time.

For the purposes of this example, the therapy device can be set to electromagnetic radiation in the range of about 380 to about 460 nm, more preferably in the range of about 395 to about 430 nm, and even more preferably in the range of about 405 to about 425 nm. The electromagnetic radiation can be about 415 nm. The device can alternatively or additionally comprise electromagnetic radiation in the range of about 460 to about 900 nm, more preferably in the range of about 550 to about 900 nm, and even more preferably in the range of about 570 to about 850 nm. The electromagnetic radiation can be about 630 nm.

The method for treating acne using to the present invention wherein material 38 comprises a desired amount of benzoyl peroxide and/or salicylic acid can be carried out about once a week, about once a day, or multiple times a day. Most preferably, the methods are carried out about once a day. The energy source can be applied to each section of the skin to be treated for a duration in the range of about 10 seconds to about 60 minutes, more preferably in the range of about 30 seconds to about 30 minutes, and even more preferably in the range of about 60 seconds to about 10 minutes each time the method is carried out. The duration can be about 90 seconds long. The dose received by each section of the skin to be treated can be in the range of about 5 Joules to about 60 Joules, more preferably in the range of about 10 Joules to about 50 Joules, and more preferably in the range of about 20 Joules to about 40 Joules. The dose can be about 30 Joules. The treatment can be applied for a period of about 1 to about 12 weeks, more preferably in the range of about 3 weeks to about 10 weeks, and most preferably in the range of about 6 weeks to about 8 weeks depending on the need of the individual.

However, depending on the severity and/or nature of the ailment and particular properties of the user's skin, much less time and/or total dose can be required.

It will be understood that other medicaments, active ingredients, or supplements can be used for the treatment or prevention of other indications using the device and material described herein.

It will be understood that medicaments, active ingredients, or supplements currently used, when used in conjunction with the device of the present invention, can demonstrate an increase in effectiveness and therefore less of the medicament, active ingredient, or supplement and/or a shorter treatment time can be required to achieve a desirable result. For example, blue light (between 405 to 425 nanometers) can be absorbed by the skin and warm the skin sufficiently to increase the effectiveness of peroxide used for the treatment or prevention of acne. In such a case, the amount of peroxide or the duration of application of the peroxide currently recommended can be advantageously decreased. The blue light can also provide a synergistic effect by also being detrimental to the survival of any acne-causing bacteria residing on the skin. As is known in the art, P. acnes absorbs light from the ultraviolet region to about 430 nm, and also absorbs light at about 630 nm. Blue light phototherapy works for a majority of patients with P. Acne vulgaris. The bacteria is made up of an endogenous porphyrin which is a naturally occurring photosensitizer. This photosensitizer absorbs the blue light energy between about 405 to about 425 nanometers and forms a singlet oxygen which simply destroys the bacteria cell. Light administered to the skin at these wavelengths is usually absorbed by the epidermis and can penetrate it to a depth of at least about 1 mm.

The device of the present invention can also be used for photorejuvenation therapy. Photorejuvenation therapy can involve, for example, using the device with electromagnetic radiation including dominant emissions in the range of about 500 nm to about 1000 nm, more preferably in the range of about 550 nm to about 900 nm and even more preferably in the range of about 570 nm to about 650 nm. Most preferably dominant electromagnetic emissions are used at about 580 nm, about 630 nm, about 633 nm, about 660 nm, and/or emissions from in the range of about 800 nm to about 900 nm. The device can be further adapted to provide ultrasound or microwave energy which can have the effect of reducing inflammation, promoting cell repair, decreasing the appearance of fine lines and wrinkles, reducing pore size, reducing redness and improving skin texture. Alternatively, such ultrasound or microwave energy can be applied separately from the application of the device.

The device of the present invention can also be used to treat cellulite. The treatment of cellulite can involve, for example, using the device with electromagnetic radiation including dominant emissions in the range of about 500 nm to about 900 nm, more preferably in the range of about 550 nm to about 800 nm and even more preferably in the range of about 650 nm to about 750 nm. Other preferred electromagnetic radiation sources to treat cellulite include laser energy or LED energy at about 810 nm, or a combination of radiofrequency and infrared radiation. It will be noted that if laser energy is used, the treatment should be supervised by a medical professional. The length of the treatments for cellulite can be in the range of about 10 seconds to about 180 minutes, more preferably in the range of about 20 sconds to about 60 minutes, and even more preferably in the range of about 30 seconds to about 10 minutes.

Depending on the ailment under treatment, parameters such as the frequency of treatments, the number of repetitions, and the duration of pulses can be adjusted so that the patient receives a total dose in the treated section of the skin of, for example, in the range of about 50 milliJ to about 100 J, more preferably in the range of about 500 milliJ to about 80 J and even more preferably in the range of about 1 J to about 50 J.

It will be recognized by those skilled in the art that the amount of pigment in a user's skin can affect the duration and/or intensity required for treatments using the device and material of the present invention. For example, the amount of pigment in skin is directly proportional to absorption of light at the surface of the skin. Therefore, there can be more absorption of light at the surface of darker skin types at depths of, for example, about 1-2 mm, and less penetration of light to depths of, for example, about 3-4 mm, as compared to fairer skin types. For treatments where deeper penetration is desired, duration and/or intensity of treatments can have to be increased for darker skin types.

The device and materials of the present invention can be used with treatment regimens known in the art. Any adjustments required to known treatment regimens would be apparent to those skilled in the art and should not require undue experimentation. The device and materials of the present invention can be used to treat such ailments as acne, arthritic pain, chronic pain, carpel tunnel syndrome, cellular damage, soft tissue injury, TMJ, diabetic neuropathy, neuralgia, aging skin, eczema, rosacia, actinic keratoses, seasonally affected disorder, inflammation, fine lines and wrinkles, cellulite, mucositis (oral mucosa), psoriasis, oral candida, oral cancer, wounds, soft tissue injuries such as capsulitis, bursitis, sprains, strains, hematomas and tendinitis, acute and chronic joint problems such as osteoarthritis, rheumatoid arthritis and ligament and tendon injuries, tendinitis, chronic pain such as post herpetic neuralgia, chronic back and neck pain, metatarsalgia, trigeminal neuralgia, brachial neuralgia, plantar fasciitis, and cellular damage.

Therapy using electromagnetic radiation can also be used to treat non-union and small bone fractures, herpes, apthous ulcers, leg ulcers, dermatitis, wound healing, bums, acute epididymitis, otorhinolaragngology, gynecology, obstetrics, superficial AP stimulation and tonification, cosmetic imperfections, among other things.

The device and materials of the present invention can be used to generally improve the appearance of skin. Any improvement of the appearance of skin can be temporary or somewhat permanent and can be measured in such terms as skin glow, clarity, texture, and smoothness.

The above detailed description is of the best presently contemplated mode of carrying out the invention. This description is not to be taken in a limiting sense, but is made merely for the purpose of illustrating the general principles of the invention. The scope of the invention is best defined by the appended claims.

In yet other variations, the contents of EEPROM 312 can be used for updating database 200. As an example, database 200 can be maintained in a separate persistent storage device such as Flash RAM or a hard drive included in device 20. Accordingly, persistent storage device can be updated with the contents of EEPROM 312, once faceplate 23 including EEPROM 312 is attached to device 20.

In yet other variations, control mechanism 300 can be implemented using different elements. For example, operations in control mechanism 300 can be carried out using an analog control circuit. In yet other variations, a programmable logic array (PLA) or a custom designed processor can be used as a processor 44. In further variations, other types of controller can be used as processor 44. In yet other variations, flash memory 304 can be replaced by a non volatile storage device such as an EEPROM, a read only memory (ROM), or a hard drive. In yet other variations, flash memory could be used in place of RAM 308. Moreover, storage devices included, such as EEPROM, ROM, RAM, hard drives, Flash RAM and others, could be removable such that the storage devices can be exchanged for updating the information accessible to device 20. In other variations, power driver 344 can be part of control mechanism 300. Moreover, different input and output devices can be used in place of a switch 35, power LED 316 and Speaker 320. For example, different types of lights or multiple lights can be used in place of power LED 320. Speaker 320 can be replaced with a vibrator, or other device capable of getting a user's attention. Switch 35 can take the form of a push button switch or a touch sensitive switch.

In other variations, record 204 can be accessed directly from EEPROM 312 or flash-memory 304 during the operation of device 20, without the need to move the data into RAM 308. In yet other variations, only a portion of the data corresponding to a record or multiple records can be present in EEPROM 312, the remainder being contained in database 200. In further variations, a control mechanism can also reside on faceplate 23, allowing the performance of method 400 or part thereof on faceplate 23.

In other variations, Method 400 can be altered such that different number of beeps, and light signals and wait times are used for informing the user of warnings and alarms. For example, additional LEDs can be used in place of sound alarms. Or different types of lights and colors can be used. Intensity, instead of color of LEDs can be altered or different color changes can be used. Duration of waits times can also vary.

## Claims

1. A therapy device for delivering electromagnetic radiation to the skin of a user, comprising:
a) a head (22) having at least one faceplate (23) for application to the skin;
b) an energy source (30) housed within the head, for emitting a desired wavelength of electromagnetic radiation;
c) a material dispensing system (340) for dispensing a desired material (38) for use with the device;
d) a temperature sensing device (332) for delivering temperature data to said control mechanism wherein the control mechanism can adjust the energy source when the received temperature data is outside pre-specified limits; and
e) a control mechanism (34) for controlling the energy source and the material dispensing system, wherein the control mechanism includes a processor (44) and a storage device (308); the storage device being operable to maintain regimen data; the processor being operable to control the energy source and the material dispensing system in accordance with the regimen data; and
wherein the faceplate (23) is removable and interchangeable and includes an identification device that communicates the identity of the faceplate to the control mechanism (34).

2. The device of claim 1, wherein the control mechanism is operable to control the emission of radiation from the energy source at a power level and for a period of time in accordance with the regimen data.

3. The device of claim 1, further comprising an interface (70) operable to conduct communications with a computing device for updating the regimen data.

4. The device of claim 1, further comprising an interface operable to conduct communications with a computer network having at least one server for updating the regimen data.

5. The device of claim 1, wherein the storage device is removable.

6. The device of claim 1, wherein said device is further operable to maintain, in said storage device, logging data representing a usage of the therapy device; said device further comprising an interface operable to conduct communications with a computing device for uploading the logging data.

7. The device of claim 1, wherein the energy source is a light source.

8. The device of claim 1, wherein the energy source is selected from the group consisting of electrodeless lamps, microwaves, fluorescent tube, quartz halogen lamp, arc lamp, laser, laser diode, and light emitting diode.

9. The device of claim 1 wherein the energy source has at least one peak wavelength selected from about 410, 415, 580, 630, 660, 663, 680, 800, 810, 820, 830, 840, 850, and 900 nm.

10. The device of claim 1 wherein the energy source comprises at least one peak wavelength having a band width of about 40 nm, more preferably about 20 nm.

11. The device of claim 1 wherein the energy source has a peak wavelength of about 415 nm and a bandwidth of about 20 nm.

12. The device of claim 1 wherein the energy source operates continuously or in a pulsed manner when activated.

13. The device in claim 1 wherein the faceplate is integral to the structure of the head.

14. The device of claim 1 wherein the dispensing system is adapted to receive a removable container (36) for containing the material.

15. The device as claimed in claim 14, wherein the container has an identifier that is readable by the device.

16. The device of claim 1, wherein the faceplate includes an extension that is functionally connected to the head.

17. The device of claim 1, further comprising an energy reflective layer disposed on the head.

18. The device of claim 1, wherein the temperature sensing device is a p-n junction diode.

19. The device of any preceding claim, further comprising a material comprising a medicament, active ingredient, or supplement.

20. The device of claim 19, wherein the index of refraction of the material is approximately 1.5.

21. The device of claim 19 or 20, wherein the medicament, active ingredient, or supplement is selected from the group consisting of aloe, Vitamin E, a hydration agent, Vitamin C, Vitamin D, Vitamin A, Vitamin K, Vitamin F, Retin A (Tretinoin), Adapalene, Retinol, Hydroquinone, Kojic acid, a growth factor, echinacea, an antibiotic, an antifungal, an antiviral, a bleaching agent, an alpha hydroxy acid, a beta hydroxy acid, salicylic acid, antioxidant triad compound, a seaweed derivative, a salt water derivative, algae, an antioxidant, a phytoanthocyanin, phthalocyanine, a phytonutrient, plankton, a botanical product, a herbaceous product, a hormone, lanolin, an enzyme, a mineral, a genetically engineered substance, a cofactor, a catalyst, an antiaging substance, insulin, trace elements (including ionic calcium, magnesium, etc.), Coenzyme Q10, minerals, minoxidil, a dye, a natural or synthetic melanin, a metalloproteinase inhibitor, proline, hydroxyproline, an anesthetic substance, benzoyl peroxide, amino levulinic acid, chlorophyll, bacteriachlorophyll, neutraceuticals, cosmeceuticals, copper chlorophyllin, chloroplasts, carotenoids, phycobilin, rhodopsin, anthocyanin, and derivatives, subcomponents, immunological complexes and antibodies directed towards any component of the target skin structure or apparatus, and analogs of the above items both synthetic and natural, as well as combinations thereof.

22. The device of claim 19 or 20, wherein the medicament, active ingredient, or supplement is for the treatment selected from a group consisting of anti-aging treatment, photorejuvenation treatment, cosmetic treatment, acne treatment, cancer treatment, cellulite treatment, and photodamage treatment.

23. The device of claim 22, wherein the medicament, active ingredient, or supplement is benzoyl peroxide.

24. The device of claim 22, wherein the medicament, active ingredient or supplement is salicylic acid.

25. Use of the device according to any preceding claim for improving the appearance of the skin of a user.

26. The device according to any of claims 1 to 24, wherein the faceplate comprises a substrate (57) for supporting the energy source;
at least one aperture (28) in said substrate for passing the material therethrough;
a mount (25) for attaching said substrate to the body; said body housing the control mechanism for controlling said energy source and a container for storing said material.

27. The device of claim 26, further comprising a connector disposed within said substrate that communicates with said control mechanism when the mount is attached to said body.

28. The device of claim 26, wherein the connector transmits power from a power source in said body to said energy source.

29. The device of claim 26, wherein the faceplate further comprises the memory storage device for storing regimen data.

30. The device of claim 26, wherein the faceplate further comprises an outer surface affixed generally parallel to the substrate with said energy source disposed therebetween, said outer surface defining at least one aperture for passing the material therethrough.

31. The device according to claim 1, wherein the identity of the faceplate (23) is contained on the faceplate as an identifier readable by a sensor located on the device (20).

## Patentansprüche

1. Ein Therapiegerät, das der Haut eines Anwenders elektromagnetische Strahlung zuführt, umfassend:
a) einen Kopfteil (22) mit wenigstens einer Frontplatte (23) zur Anwendung auf der Haut,
b) eine im Kopfteil eingebaute Energiequelle (30), die elektromagnetische Strahlung in einer gewünschten Wellenlänge aussendet,
c) ein Materialabgabesystem (340), das ein gewünschtes Material (38) abgibt, zur Verwendung mit dem Gerät,
d) einen Temperaturfühler (332), der Temperaturdaten an den Steuermechanismus sendet, wobei der Steuermechanismus die Energiequelle einstellen kann, wenn die empfangenen Temperaturdaten außerhalb vorbestimmter Grenzen liegen, und
e) einen Steuermechanismus (34) zur Steuerung der Energiequelle und des Materialabgabesystems, wobei der Steuermechanismus einen Prozessor (44) und eine Speichereinrichtung (308) enthält, wobei die Speichereinrichtung in der Lage ist, Behandlungsprogrammdaten zu speichern, der Prozessor in der Lage ist, die Energiequelle und das Materialabgabesystem nach Maßgabe der Behandlungsprogrammdaten zu steuern, und
wobei die Frontplatte (23) abnehmbar und auswechselbar ist und eine Kennzeichnung enthält, die die Identität der Frontplatte an den Steuermechanismus (34) übermittelt.

2. Das Gerät nach Anspruch 1, wobei der Steuermechanismus in der Lage ist, nach Maßgabe der Behandlungsprogrammdaten den Leistungspegel und die Dauer der Emission von Strahlung von der Energiequelle zu steuern.

3. Das Gerät nach Anspruch 1, ferner umfassend ein Interface (70), das in der Lage ist, mit einem EDV-Gerät zu kommunizieren, um die Behandlungsprogrammdaten zu aktualisieren.

4. Das Gerät nach Anspruch 1, ferner umfassend ein Interface, das in der Lage ist, mit einem Computernetzwerk mit wenigstens einem Server zu kommunizieren, um die Behandlungsprogrammdaten zu aktualisieren.

5. Das Gerät nach Anspruch 1, wobei der Speicher abnehmbar ist.

6. Das Gerät nach Anspruch 1, wobei das Gerät ferner in der Lage ist, im Speicher Protokolldaten zu speichern, die eine Verwendung des Therapiegeräts zeigen, wobei das Gerät ferner ein Interface umfasst, das in der Lage ist, mit einem EDV-Gerät zu kommunizieren, um die Protokolldaten hochzuladen.

7. Das Gerät nach Anspruch 1, wobei die Energiequelle eine Lichtquelle ist.

8. Das Gerät nach Anspruch 1, wobei die Energiequelle ausgewählt ist aus der Gruppe, bestehend aus elektrodenlosen Lampen, Mikrowellen, Leuchtstoffröhren, Quarzhalogenlampen, Bogenlampen, Laser, Laserdiode und Leuchtdiode.

9. Das Gerät nach Anspruch 1, wobei die Energiequelle wenigstens eine Peakwellenlänge, ausgewählt aus etwa 410, 415, 580, 630, 660, 663, 680, 800, 810, 820, 830, 840, 850 und 900 nm, besitzt.

10. Das Gerät nach Anspruch 1, wobei die Energiequelle wenigstens eine Peakwellenlänge mit einer Bandbreite von etwa 40 nm, vorzugsweise etwa 20 nm, umfasst.

11. Das Gerät nach Anspruch 1, wobei die Energiequelle wenigstens eine Peakwellenlänge von etwa 415 nm und eine Bandbreite von etwa 20 nm besitzt.

12. Das Gerät nach Anspruch 1, wobei die Energiequelle, wenn aktiviert, kontinuierlich oder gepulst arbeitet.

13. Das Gerät nach Anspruch 1, wobei der Frontplatte Bestandteil der Struktur des Kopfteils ist.

14. Das Gerät nach Anspruch 1, wobei das Abgabesystem angepasst ist, um einen abnehmbaren Behälter (36) aufzunehmen, der das Material enthält.

15. Das Gerät wie in Anspruch 14 beansprucht, wobei der Behälter eine vom Gerät lesbare Kennzeichnung besitzt.

16. Das Gerät nach Anspruch 1, wobei die Frontplatte eine Verlängerung umfasst, die funktionell mit dem Kopfteil verbunden ist.

17. Das Gerät nach Anspruch 1, ferner umfassend eine auf dem Kopfteil angeordnete energiereflektierende Schicht.

18. Das Gerät nach Anspruch 1, wobei der Temperaturfühler eine p-n-Übergang-Diode ist.

19. Das Gerät nach einem vorhergehenden Anspruch, ferner umfassend ein Material, das ein Medikament, einen Wirkstoff oder einen Zusatzstoff umfasst.

20. Das Gerät nach Anspruch 19, wobei der Brechungsindex des Materials etwa 1,5 ist.

21. Das Gerät nach Anspruch 19 oder 20, wobei das Medikament, der Wirkstoff oder der Zusatzstoff ausgewählt ist aus der Gruppe, bestehend aus Aloe, Vitamin E, einem Hydratisierungsmittel, Vitamin C, Vitamin D, Vitamin A, Vitamin K, Vitamin F, Retin A (Tretinoin), Adapalen, Retinol, Hydrochinon, Koji-Säure, einem Wachstumsfaktor, Echinacin, einem Antibiotikum, einem Antimykotikum, einem antiviralen Mittel, einem Bleichmittel, einer alpha-Hydroxysäure, einer beta-Hydroxysäure, Salicylsäure, antioxidativer Dreiergruppenverbindung, einem Tangderivat, einem Salzwasserderivat, Algen, einem Antioxidationsmittel, einem Phytoanthocyanin, Phthalocyanin, einem Phytonährstoff, Plankton, einem botanischen Produkt, einem krautigen Produkt, einem Hormon, Lanolin, einem Enzym, einem Mineral, einer genetisch veränderten Substanz, einem Cofaktor, einem Katalysator, einer Anti-Aging-Substanz, Insulin, Spurenelementen (einschließlich ionisches Kalzium, Magnesium usw.), Coenzym Q10, Mineralien, Minoxidil, einem Farbstoff, einem natürlichen oder synthetischen Melanin, einem Metalloproteinase-Inhibitor, Prolin, Hydroxyprolin, einem Anästhetikum, Benzoylperoxid, Aminolevulinsäure, Chlorophyll, Bakterienchlorophyll, Neutrazeutika, Kosmezeutika, Kupferchlorophyllin, Chloroplasten, Karotinoiden, Phycobilin, Rhodopsin, Anthocyanin und Derivaten, Subkomponenten, Immunkomplexen und Antikörpern, die gegen eine Komponente der Ziel-Hautstruktur oder des Ziel-Apparats gerichtet sind, und Analoga der obigen Stoffe, sowohl synthetisch als auch natürlich, sowie Kombinationen davon.

22. Das Gerät nach Anspruch 19 oder 20, wobei das Medikament, der Wirkstoff oder der Zusatzstoff der Behandlung dient, die ausgewählt ist aus einer Gruppe, bestehend aus Anti-Aging-Behandlung, Photorejuvenationsbehandlung, kosmetischer Behandlung, Aknebehandlung, Krebsbehandlung, Cellulitebehandlung und Behandlung von Lichtschäden.

23. Das Gerät nach Anspruch 22, wobei das Medikament, der Wirkstoff oder der Zusatzstoff Benzoylperoxid ist.

24. Das Gerät nach Anspruch 22, wobei das Medikament, der Wirkstoff oder der Zusatzstoff Salicylsäure ist.

25. Verwendung des Geräts gemäß einem vorhergehenden Anspruch zur Verbesserung des Aussehens der Haut eines Anwenders.

26. Das Gerät gemäß einem der Ansprüche 1 bis 24, wobei die Frontplatte umfasst:
ein Substrat (57) zur Aufnahme der Energiequelle,
wenigstens eine Öffnung (28) in dem Substrat, um das Material durchzuleiten,
eine Halterung (25) zum Befestigen des Substrats an dem Körper, wobei der Körper den Steuermechanismus zur Steuerung der Energiequelle und einen Behälter zur Aufbewahrung des Materials enthält.

27. Das Gerät nach Anspruch 26, ferner umfassend einen Konnektor, der sich in dem Substrat befindet und der mit dem Steuermechanismus kommuniziert, wenn die Halterung am Körper befestigt ist.

28. Das Gerät nach Anspruch 26, wobei der Konnektor Strom von einer Stromquelle in dem Körper an die Energiequelle leitet.

29. Das Gerät nach Anspruch 26, wobei die Frontplatte ferner die Speichereinrichtung zum Speichern von Behandlungsprogrammdaten umfasst.

30. Das Gerät nach Anspruch 26, wobei die Frontplatte ferner eine Außenfläche umfasst, die im Allgemeinen parallel zum Substrat angebracht ist, wobei sich die Energiequelle dazwischen befindet, und wobei die Außenfläche wenigstens eine Öffnung definiert, um Material durchzuleiten.

31. Das Gerät gemäß Anspruch 1, wobei die Identität der Frontplatte (23) als eine Kennzeichnung, die von einem auf dem Gerät (20) befindlichen Sensor gelesen werden kann, auf der Frontplatte enthalten ist.

## Revendications

1. Un dispositif thérapeutique pour délivrer un rayonnement électromagnétique sur la peau d'un utilisateur, comprenant :
a) une tête (22) ayant au moins une plaque frontale (23) pour application sur la peau ;
b) une source d'énergie (30) située dans la tête, pour émettre une longueur d'onde de rayonnement électromagnétique souhaitée ;
c) un système de distribution de matière (340) pour distribuer une matière souhaitée (38) destinée à être utilisée avec le dispositif ;
d) un capteur de température (332) pour fournir des données de température au dit mécanisme de contrôle dans lequel le mécanisme de contrôle peut ajuster la source d'énergie quand les données de température reçues sont en dehors des limites pré-établies ; et
e) un mécanisme de contrôle (34) pour contrôler la source d'énergie et le système de distribution de matière, dans lequel le mécanisme de contrôle inclut un processeur (44) et un dispositif de stockage (308) ; l'appareil de stockage étant utilisable pour conserver les données de traitement ; le processeur étant utilisable pour contrôler la source d'énergie et le système de dispersion de matière en accord avec les données de traitement ; et
dans lequel la plaque frontale (23) est amovible et interchangeable et inclut un dispositif d'identification qui communique l'identité de la plaque frontale au mécanisme de contrôle (34).

2. Le dispositif selon la revendication 1, dans lequel le mécanisme de contrôle est utilisable pour contrôler l'émission de rayonnement de la source d'énergie à un niveau de puissance et pour une période de temps conformément aux données de traitement.

3. Le dispositif selon la revendication 1, comprenant également une interface (70) utilisable pour assurer les communications avec un appareil informatique pour mettre à jour les données de traitement.

4. Le dispositif selon la revendication 1, comprenant également une interface utilisable pour assurer les communications avec un réseau informatique ayant au moins un serveur pour mettre à jour les données de traitement.

5. Le dispositif selon la revendication 1, dans lequel le dispositif de stockage est amovible.

6. Le dispositif selon la revendication 1, dans lequel ledit dispositif est également utilisable pour maintenir, dans ledit dispositif de stockage, l'enregistrement des données représentant une utilisation du dispositif thérapeutique ; ledit dispositif comprenant également une interface utilisable pour assurer les communications avec un appareil informatique pour télécharger les données d'enregistrement.

7. L'appareil selon la revendication 1, dans lequel la source d'énergie est une source de lumière.

8. L'appareil selon la revendication 1, dans lequel la source d'énergie est choisie dans le groupe constitué des lampes sans électrodes, micro-ondes, tube fluorescent, lampe quartz-halogène, lampe à arc, laser, diode laser, et diode lumineuse.

9. Le dispositif selon la revendication 1 dans lequel la source d'énergie a au moins un pic de longueur d'onde choisi d'environ 410, 415, 580, 630, 660, 663, 680, 800, 810, 820, 830, 840, 850, et 900 nm.

10. Le dispositif selon la revendication 1 dans lequel la source d'énergie comprend au moins un pic de longueur d'onde ayant une largeur de bande d'environ 40 nm, plus préférentiellement environ 20 nm.

11. Le dispositif selon la revendication 1 dans lequel la source d'énergie a un pic de longueur d'onde d'environ 415 nm et une largeur de bande d'environ 20 nm.

12. Le dispositif selon la revendication 1 dans lequel la source d'énergie fonctionne continuellement ou par impulsion quand elle est activée.

13. Le dispositif selon la revendication 1 dans lequel la plaque frontale forme une partie intégrale de la structure de la tête.

14. Le dispositif selon la revendication 1 dans lequel le système de distribution est adapté pour recevoir un récipient amovible pour contenir la matière.

15. Le dispositif tel que revendiqué dans la revendication 14, dans lequel le récipient a un identifiant qui est lisible par le dispositif.

16. Le dispositif selon la revendication 1, dans lequel la plaque frontale inclut une extension qui est connectée de façon fonctionnelle à la tête.

17. Le dispositif selon la revendication 1, comprenant également une couche réfléchissant l'énergie disposée sur la tête.

18. Le dispositif selon la revendication 1, dans lequel le capteur de température est une diode à jonction p-n.

19. Le dispositif selon l'une quelconque des précédentes revendications comprenant également une matière contenant un médicament, une substance active, ou un complément.

20. Le dispositif selon la revendication 19, dans lequel l'index de réfraction de la matière est approximativement 1.5

21. Le dispositif selon la revendication 19 ou 20, dans lequel le médicament, la substance active, ou le complément est choisi dans le groupe constitué par l'Aloe, la Vitamine E, un agent d'hydratation, la Vitamine C, la Vitamine D, la Vitamine A, la Vitamine K, la Vitamine F, la Retine A (Tretinoine), l'Adapalene, le Rétinol, l'Hydroquinone, l'Acide kojique, un facteur de croissance, l'Echinacea, un antibiotique, un antifongique, un antiviral, un agent de blanchiment, un acide alpha-hydroxylé, un acide beta-hydroxylé, l'acide salicylique, un composé tri-antioxydant, un dérivé d'algue, un dérivé d'eau salée, une algue, un antioxydant, un phytoanthocyanine, un phthalocyanine, un phytonutriment, un plancton, un produit botanique, un produit herbacé, une hormone, la lanoline, une enzyme, un minéral, une substance génétiquement modifiée, un cofacteur, un catalyseur, une substance anti-âge, l'insuline, un oligo-éléments (incluant le calcium ionique, magnésium, ...), le Coenzyme Q10, les minéraux, le monoxidil, un colorant, une mélanine naturelle ou synthétique, un inhibiteur de métalloprotéinase, la proline, l'hydroxyproline, une substance anesthésique, le peroxyde de benzoyle, l'acide aminolevulinique, la chlorophylle, la chlorophylle bactérienne, les neutracétiques, les cosméceutiques, le cuivre, la chlorophylline, les chloroplastes, les caroténoïdes, la phycobiline, la rhodopsine, l'anthocyanine et dérivés, des sous-éléments, des complexes immunologiques, et des anticorps dirigés contre l'un quelconque des composants de la structure de la peau ou de l'appareil cible, et les analogues des éléments mentionnés précédemment, tant naturels que synthétiques, ainsi que les combinaisons de ceux-ci.

22. Le dispositif selon l'une quelconque des revendications 19 ou 20, dans lequel le médicament, la substance active, ou le complément est pour le traitement choisi dans le groupe constitué par un traitement anti-âge, un traitement de photo-rajeunissement, un traitement cosmétique, un traitement de l'acné, un traitement du cancer, un traitement de la cellulite, et un traitement des peaux lésées par le soleil.

23. Le dispositif selon la revendication 22, dans lequel le médicament, la substance active, ou le complément est le peroxyde de benzoyle.

24. Le dispositif selon la revendication 22, dans lequel le médicament, la substance active ou le complément est l'acide salicylique.

25. Utilisation du dispositif selon l'une quelconque des précédentes revendications pour améliorer l'apparence de la peau de l'utilisateur.

26. Le dispositif selon l'une quelconque des revendications 1 à 24, dans lequel la plaque frontale comprend un support (57) pour supporter la source d'énergie ;
au moins un orifice (28) dans ledit support pour faire passer la matière à travers ;
un matériel de fixation (25) pour attacher ledit support au corps, le mécanisme de contrôle pour contrôler ladite source d'énergie et un récipient pour stocker ladite matière étant situé dans ledit corps.

27. Le dispositif selon la revendication 26, comprenant également un connecteur disposé dans ledit support qui communique avec ledit mécanisme de contrôle quand le matériel de fixation est attaché audit corps.

28. Le dispositif selon la revendication 26, dans lequel le connecteur transmet l'alimentation depuis la source d'alimentation dans ledit corps à ladite source d'énergie.

29. Le dispositif selon la revendication 26, dans lequel la plaque frontale comprend également un dispositif de stockage de mémoire pour stocker les données de traitement.

30. Le dispositif selon la revendication 26, dans lequel la plaque frontale comprend également une surface externe apposée généralement de façon parallèle par rapport au support, avec ladite source d'énergie disposée entre les deux, ladite surface externe définissant au moins un orifice pour faire passer la matière à travers.

31. Le dispositif selon la revendication 1, dans lequel l'identité de la plaque frontale (23) figure sur la plaque frontale comme un identifiant lisible par un capteur situé sur l'appareil (20).
